# FASCICULE DE BREVET EUROPEEN

(11) **EP 0 743 300 B1**
(45) Date de publication et mention de la délivrance du brevet: **04.08.1999**
(21) Numéro de dépôt: 96400949.2
(22) Date de dépôt: 03.05.1996
(51) Int. Cl.: C07C 279/12, C07C 279/14, A61K 31/155, C07C 215/14

(54) **Analogues de la 15-déoxyspergualine, leur procédé de préparation et leur utilisation en thérapeutique**
15-Deoxyspergualinanaloge Verbindungen, Verfahren zu ihrer Herstellung und ihre Verwendung in der Therapie
15-Deoxyspergualine-related compounds, process for their preparation and their application in therapy

(30) Priorité: 17.05.1995 FR 9505862
(43) Date de publication de la demande: 20.11.1996
(73) Titulaire: FOURNIER INDUSTRIE ET SANTE, F-75008 Paris (FR)
(72) Inventeur: Lebreton, Luc, 21000 Dijon (FR); Renaut, Patrice, 21121 Hauteville- Lès-Dijon (FR); Dumas, Christine, 78000 Versailles (FR)
(74) Mandataire: Clisci, Serge

(56) Documents cités:
- EP-A- 0 105 193
- EP-A- 0 394 684
- EP-A- 0 600 762
- EP-A- 0 669 316

## Description

La présente invention concerne des nouveaux composés dont la structure reste apparentée à la 15-déoxyspergualine. Elle concerne également leur procédé de préparation et leur utilisation en thérapeutique, notamment en tant qu'agents immunosuppresseurs.

### Art antérieur

On sait que la 15-déoxyspergualine (DSG), qui a pour formule développée : 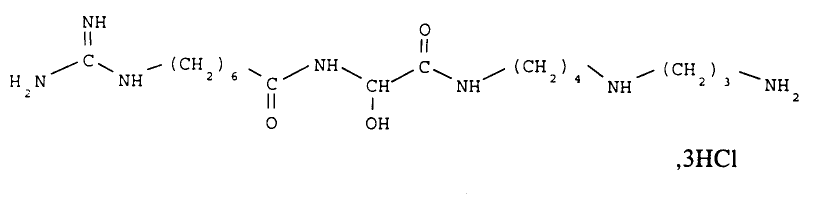 et qui est également connue sous la dénomination commune internationale "Gusperimus", possède une activité intéressante dans le domaine de l'immunosuppression. De nombreuses publications font état de cette activité : on trouvera notamment une série d'articles sur ce sujet dans "Immunomodulating Drugs" - Annals of the New York Academy of Sciences, Vol 685, pages 123 à 201.

Cependant, la 15-déoxyspergualine ne présente pas une stabilité chimique satisfaisante et on a cherché à obtenir des composés plus stables, par exemple (i) en remplaçant le groupe α-hydroxyglycine de la 15-déoxyspergualine par divers α-ou ω-aminoacides, (ii) en modifiant la structure du chaînon central, ou encore (iii) en modifiant le chaînon porteur de la fonction guanidine. Des exemples de telles modifications sont décrits dans EP-A-0 181 592, EP-A-0 105 193 et FR-A-2 698 628.

Les modifications portant sur le reste hydroxyglycine ont été reportées essentiellement dans J. Antibiot. 38 : 886-898 et J.Antibiot. 41 : 1629-1643. Selon ces publications, aucune des structures proposées ne permettait d'obtenir une activité supérieure à celle de la DSG. De même, les modifications apportées au reste spermidine, essentiellement publiées dans J. Antibiot. 40 : 1303-1315, ont conduit la plupart du temps à une perte d'activité et la présence de l'enchaînement spermidine paraissait indispensable pour obtenir un composé actif.

Or, on vient maintenant de trouver que des composés de structure apparentée à la DSG, mais ne comprenant plus l'enchaînement hydroxy-glycine et l'enchaînement spermidine, avaient cependant une activité dans le domaine de l'immunosuppression et que cette activité pouvait être supérieure à celle de la DSG.

### Objet de l'invention

La présente invention propose des composés nouveaux, dont la structure générale reste apparentée à celle de la 15-déoxyspergualine, qui sont chimiquement stables et qui présentent une activité supérieure à celle des produits connus de l'art antérieur, notamment dans le domaine de l' immunosuppression.

Les composés selon l'invention se distinguent des produits connus de l'art antérieur essentiellement par une modification importante de la partie centrale de la molécule qui conduit à un groupement de type carbamate. De plus à la différence de tous les produits connus de l'art antérieur apparentés à la 15-déoxyspergualine, les composés selon l'invention ne font plus appel à un enchaînement polyamine de type spermidine, mais à un groupe de type aminoalcool.

Les composés selon l'invention sont caractérisés en ce qu'ils sont choisis parmi l'ensemble constitué par :
**(i)** les composés de formule : 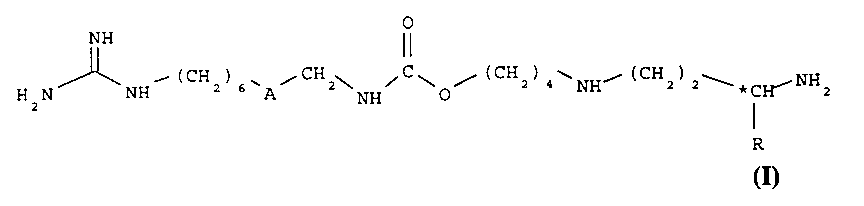 dans laquelle :
   - A: représente un groupe -CO-NH ou un groupe -NH-CO-,
   - R: représente un atome d'hydrogène ou un groupe méthyle,
   - *C,: lorsque R n'est pas l'atome d'hydrogène, est un carbone asymétrique dont la configuration peut être indéterminée (R,S) ou déterminée (R), et
**(ii)** leurs sels d'addition.

Selon l'invention, on préconise également un procédé de préparation des composés de formule I et de leurs sels d'addition, ledit procédé comprenant la déprotection d'un composé de formule : 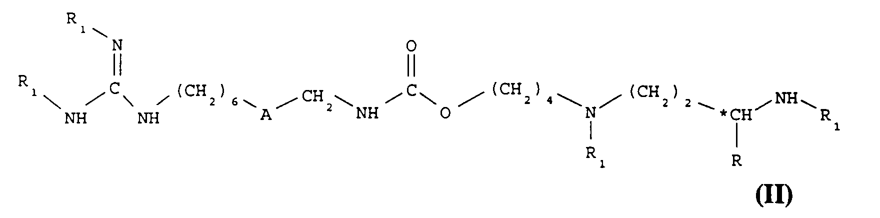 dans laquelle :
- A: représente un groupe -NH-CO- ou un groupe -CO-NH-,
- R: représente un atome d'hydrogène ou un groupe méthyle,
- *C: représente, lorsque R n'est pas l'atome d'hydrogène, un carbone asymétrique de configuration (R,S) ou de configuration (R),
l'un au moins des substituants R₁ représente un groupe protecteur de la fonction amine, les autres représentent un atome d'hydrogène,
selon un traitement réactionnel connu de l'homme de l'art, pour obtenir la déprotection des fonctions amines protégées par des groupes aminoprotecteurs R₁, et le remplacement de tous les groupes amino-protecteurs R₁ par un atome d'hydrogène.

On préconise également l'utilisation d'une substance choisie parmi les composés de formule I, leurs sels d'addition non-toxiques et leurs mélanges pour l'obtention d'un médicament destiné à une utilisation en thérapeutique pour le traitement ou la prévention des désordres immunitaires ou du paludisme, ou pour une utilisation en tant que réactif pharmacologique .

### Description détaillée de l'invention

Par "sels d'addition", on entend ici les sels d'addition d'acide obtenus par réaction d'un composé de formule I avec un acide minéral ou un acide organique. Les acides minéraux préférés pour la salification sont les acides chlorhydrique, bromhydrique, phosphorique et sulfurique. Les acides organiques préférés pour la salification sont les acides fumarique, maléique, méthanesulfonique, oxalique, citrique et trifluoroacétique.

Comme indiqué dans la formule I, les composés selon l'invention comportent un carbone noté *C qui, lorsque R n'est pas un atome d'hydrogène, est un carbone asymétrique. Quand R n'est pas un atome d'hydrogène, la présente invention englobe, parmi les composés de formule I, les composés racémiques où *C est de configuration (R,S) et l'énantiomère où *C est de configuration (R), selon les règles de détermination de structure décrites par Cahn, Ingold et Prelog. Lorsque R est H, l'atome de carbone noté *C n'est pas asymétrique.

De façon pratique, on préfère, parmi les composés de formule I comportant un carbone *C asymétrique, ceux dans lesquels ledit carbone est de configuration (R).

Les composés de formule I peuvent être obtenus selon des méthodes connues en soi, par application de mécanismes réactionnels classiques, notamment des réactions permettant d'obtenir des groupements de type carbamate.

Le procédé de préparation des composés de formule I que l'on préconise selon l'invention comprend, comme indiqué ci-dessus, la déprotection d'un composé de formule II. De façon pratique, le ou les groupes R₁, qui vont dans la réaction être remplacés par des atomes d'hydrogène, sont des groupes amino-protecteurs de type connu, notamment dans la chimie des peptides, pour bloquer temporairement les fonctions "amine" non totalement substituées.

Parmi les groupes qui conviennent à cet effet, on peut utiliser :
**(a)** des groupes de type oxycarbonyle, comme par exemple les groupes alkoxycarbonyle ou benzyloxycarbonyle :
   - Boc :: t-butyloxycarbonyle (ou 1,1-diméthyléthoxycarbonyle)
   - Fmoc :: 9-fluorénylméthyloxycarbonyle
   - Z :: benzyloxycarbonyle (ou phénylméthoxycarbonyle)
   - Z(p-Cl) :: 4-chlorobenzyloxycarbonyle
   - Z(p-OMe) :: 4-méthoxybenzyloxycarbonyle
**(b)** des groupes de type benzyle comme par exemple le groupe phenylméthyle (Bn).

Parmi ces groupes amino-protecteurs, le groupe préféré selon l'invention est le groupe Boc.

Le procédé de préparation d'un composé de formule I ou de l'un de ses sels d'addition est caractérisé en ce qu'il comprend les étapes consistant à :
**(i)** déprotéger un composé de formule : 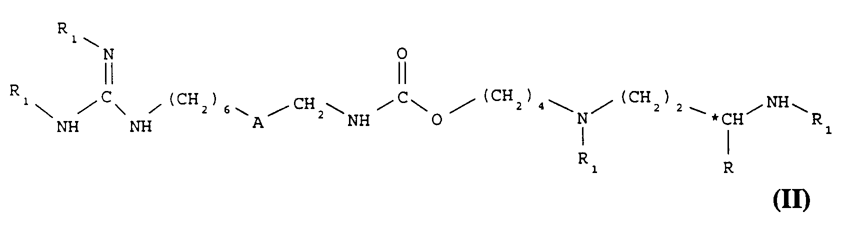 dans laquelle :
   - R: représente un atome d'hydrogène ou un groupe méthyle,
   - A: représente un groupe -CO-NH- ou un groupe -NH-CO-,
   - *C: représente, lorsque R n'est pas l'atome d'hydrogène, un carbone asymétrique de configuration indéterminée (R,S) ou de configuration déterminée (R),
   l'un au moins des substituants R₁ représente un groupe protecteur d'amine de type oxycarbonyle ou benzyle, et les autres substituants R₁, s'il y a lieu, représentent un atome d'hydrogène,
   selon un traitement adapté à la nature du groupe amino-protecteur, comme par exemple, si ce groupe amino-protecteur est un groupe de type alcoxycarbonyle, par action d'un acide fort tel que l'acide trifluoroacétique ou, si ce groupe amino-protecteur est un groupe de type benzyle, par hydrogénation catalytique en présence d'un catalyseur à base de palladium, pour obtenir un composé de formule I sous forme de base libre ou de l'un de ses sels d'addition et, si nécessaire,
**(ii)** à partir de la base libre ou de son sel d'addition obtenu selon l'étape (i), obtenir les autres sels d'addition. En pratique à l'étape (ii), à partir d'un sel d'addition, on obtient les autres sels d'addition, soit par l'intermédiaire du composé de formule I sous forme de base libre, soit par échange du contre-ion en présence d'un excès de l'acide correspondant au sel à obtenir.

Pour la préparation d'un composé de formule II, on peut mettre en oeuvre un procédé choisi parmi les variantes suivantes :
**(a)** la variante A qui comprend les étapes consistant à :
   **(i)** condenser un alcool de formule : 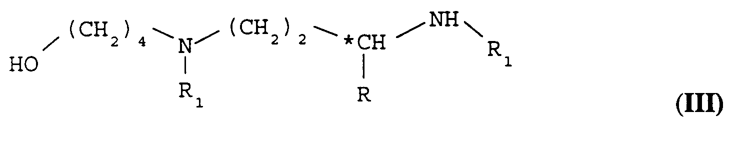 dans laquelle :
      - R: représente un atome d'hydrogène ou un groupe méthyle,
      - R₁: représente un groupe amino-protecteur comme par exemple un groupe Boc,
      - *C: représente, lorsque R n'est pas l'atome d'hydrogène, un carbone asymétrique de configuration (R,S) ou (R),
      avec un chloroformiate ou un carbonate symétrique comme par exemple le chloroformiate de 4-nitro-phényle, en présence d'une base comme par exemple de la triéthylamine, dans un solvant inerte, et à la température ambiante (15-25° C), puis
   **(ii)** faire agir sur le composé obtenu au stade (i), une amine de formule : 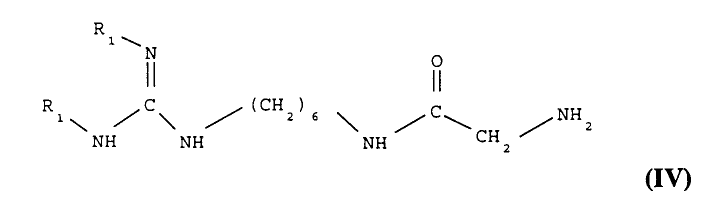 dans laquelle R₁ représente un groupe amino-protecteur, par exemple un groupe Boc, dans un solvant inerte, à une température d'environ 25 à 50° C, pour obtenir un composé de formule : 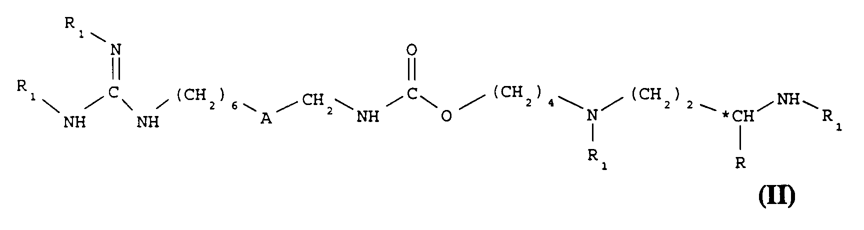 dans laquelle :
      - A: représente le groupe -NH-CO-,
      - R: représente un atome d'hydrogène ou un groupe méthyle,
      - *C: représente, lorsque R n'est pas l'atome d'hydrogène, un carbone asymétrique de configuration (R,S) ou (R),
      - R₁: représente un groupe amino-protecteur, par exemple un groupe Boc;
**(b)** la variante B qui comprend les étapes consistant à :
   **(i)** faire réagir un acide de formule : 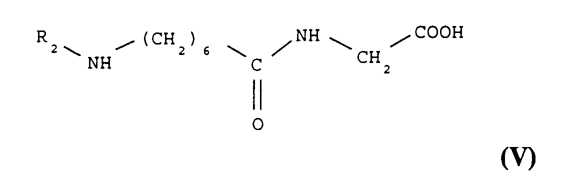
      dans laquelle R₂ représente un groupe amino-protecteur, par
      exemple un groupe benzyloxycarbonyle, avec de l' azoture de diphénylphosphoryle de formule : 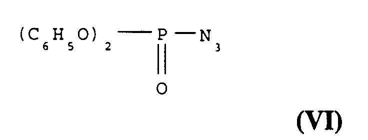 en présence d'une base telle que notamment la triéthylamine, dans un solvant comme par exemple le tétrahydrofuranne et à la température ambiante, pour obtenir un composé intermédiaire de formule 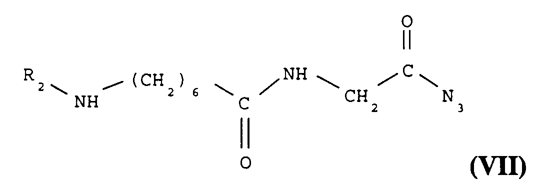
   **(ii)** faire subir au composé VII obtenu ci-dessus un réarrangement connu sous le nom de réaction de Curtius et simultanément faire réagir l'isocyanate ainsi obtenu avec un alcool de formule : 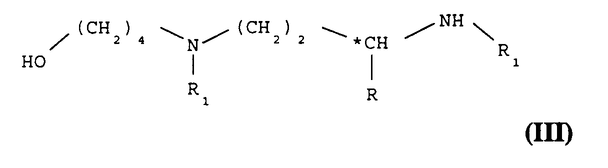 dans laquelle :
      - R: représente un atome d'hydrogène ou un groupe méthyle,
      - R₁: représente un groupe amino-protecteur différent du groupe R₂ ci-dessus, par exemple un groupe Boc,
      - *C: représente, lorsque R n'est pas l'atome d'hydrogène, un carbone asymétrique de configuration (R,S) ou (R),
      dans un solvant, tel que notamment le toluène, à une température d'environ 80 à 140° C, pendant 5 à 50 heures pour obtenir un composé de formule : 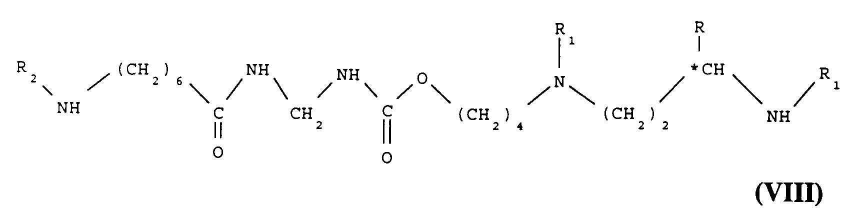
      dans laquelle R, R₁, R₂ et *C conservent les mêmes
      significations que ci-dessus,
   **(iii)** déprotéger le composé VIII obtenu ci-dessus, selon un traitement spécifique au remplacement du groupe amino-protecteur R₂ par un atome d'hydrogène, par exemple, si R₂ est un groupe benzyloxycarbonyle, en effectuant une hydrogénation catalytique en présence d'un catalyseur à base de palladium, pour obtenir un composé de formule : 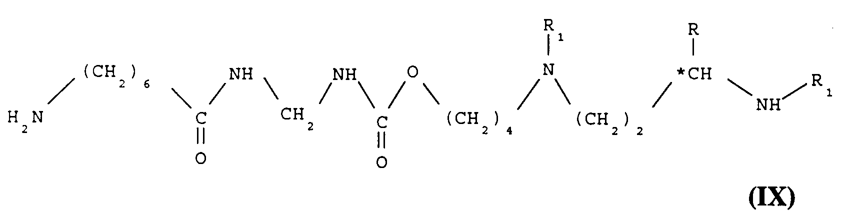
      dans laquelle R, R₁ et *C conservent les mêmes significations que ci-dessus,
   **(iv)** faire réagir le composé de formule IX obtenu au stade (iii) avec l'acide amino-iminométhanesulfonique, dans un solvant, par exemple le méthanol, à la température ambiante (15-25° C) et pendant 8 à 50 heures, pour obtenir un composé de formule : 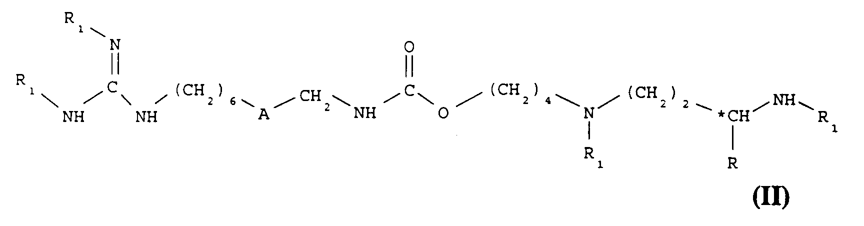 dans laquelle :
      - R et *C: conservent la même signification que ci-dessus,
      - A: représente le groupe -CO-NH-,
      - R₁: représente un groupe amino-protecteur, par exemple le groupe Boc, exception faite des deux groupes R₁ portés par la fonction guanidine qui représentent chacun un atome d'hydrogène ; ou,
   **(iv')** en variante de l'étape (iv) ci-dessus, on fait réagir le composé de formule IX avec un composé de formule : 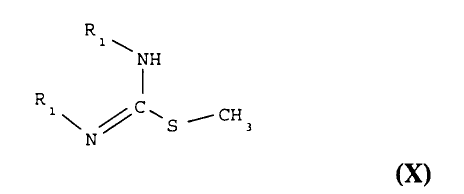 dans laquelle :
      - R₁: représente un groupe amino-protecteur comme par exemple un groupe Boc,
      dans un solvant comme par exemple du tetrahydrofuranne, en présence d'une base, notamment la triéthylamine, à la température ambiante pendant 8 à 100 heures pour obtenir un composé de formule : 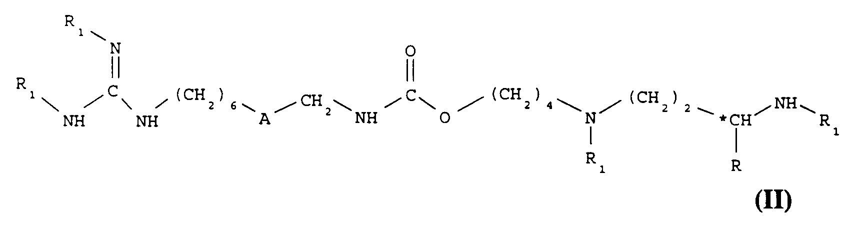 dans laquelle :
      - A: représente le groupe -CO-NH-,
      - R: représente un atome d'hydrogène ou un groupe méthyle,
      - R₁: représente un groupe amino-protecteur, par exemple un groupe Boc,
      - *C: représente, lorsque R n'est pas l'atome d'hydrogène, un carbone asymétrique de configuration (R,S) ou (R).

   Le composé de formule : 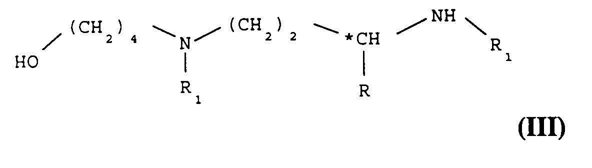 dans lequel :
   - R: représente un groupe méthyle,
   - *C: représente un atome de carbone de configuration (R,S) ou (R)
   - R₁: représente un groupe amino-protecteur, par exemple un groupe Boc,
   peut être obtenu
   **(a)** par action du 4-amino-butanol sur un composé de formule : 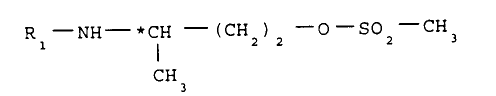 dans laquelle R₁ représente un groupe amino-protecteur, par exemple le groupe Boc,
      pour obtenir un composé de formule : 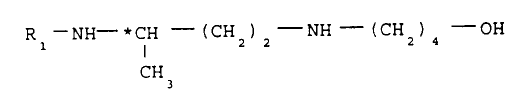 puis introduction d'un groupe amino-protecteur sur la fonction amine libre, par exemple avec du dicarbonate de t-butyle (Boc₂O) si R₁ représente le groupe Boc, et obtenir le composé de formule III attendu, ou
   **(b)** par action d'un halogénure de formule :

      X―(CH₂)₄―O―R₃

      dans laquelle X représente un halogène, par exemple un atome d'iode ou un atome de brome, et R₃ représente un groupe hydroxy-protecteur, notamment un groupe trityle,
      sur un composé de formule : 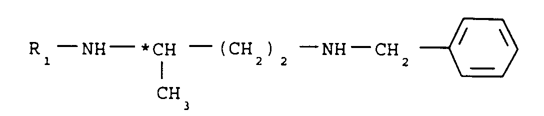 dans laquelle R₁ représente un groupe amino-protecteur, par exemple le groupe Boc, pour obtenir un composé de formule : 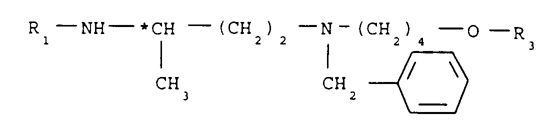 puis déprotection sélective de ce produit pour obtenir le composé de formule : 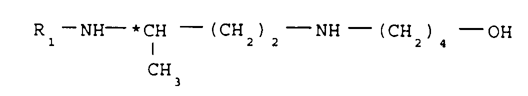 et introduction sur ce composé du groupement amino-protecteur R₁, par exemple par action du dicarbonate de t-butyle si R₁ représente le groupe Boc, pour obtenir le composé de formule III attendu.

   Les composés de formule III dans laquelle R est un groupe méthyle et R₁ est un groupe Boc sont nouveaux et constituent l'un des objets de l'invention.
   Le composé de formule V : 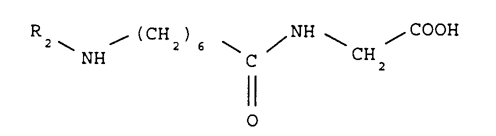 dans laquelle R₂ représente un groupe amino-protecteur, notamment le groupe Z (benzyloxycarbonyle), peut être obtenu au départ d'un composé de formule : 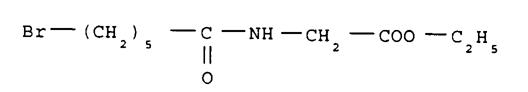 par actions successives
   **(a)** d'un cyanure qui permet d'obtenir le composé de formule : 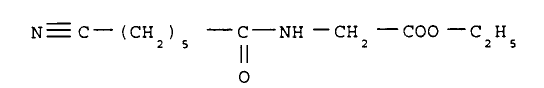
   **(b)** d'une solution de soude qui conduit à l'acide : 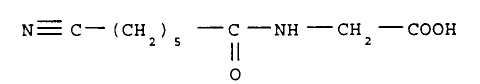
   **(c)** de l'hydrogène en présence d'un catalyseur d'hydrogénation pour obtenir l'amine : 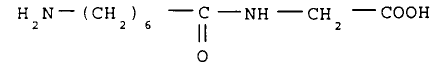
   **(d)** enfin, d'un réactif d'introduction du groupe amino-protecteur, comme par exemple du chloroformiate de benzyle, pour obtenir le composé de formule V attendu, dans laquelle R₂ représente un groupe benzyloxycarbonyle (Z).

L'invention sera mieux comprise à la lecture des exemples qui suivent et des résultats d'essais pharmacologiques obtenus avec les composés selon l'invention, comparativement aux résultats obtenus avec des produits connus de l'art antérieur. La nomenclature utilisée dans les exemples est celle préconisée par les Chemical Abstracts; ainsi un ester du type "...-oate de t-butyle" sera écrit sous la forme "acide ...-oïque, 1,1-diméthyléthyl ester".

Dans la partie expérimentale, les préparations sont relatives aux intermédiaires et les exemples sont relatifs aux produits selon l'invention.

Lorsque les composés comportent dans leur structure un carbone asymétrique, l'absence d'indication particulière ou l'indication (R,S) signifie qu'il s'agit d'un mélange sensiblement équimoléculaire des deux énantiomères (i.e. composé "racémique"). Lorsque ces mêmes composés sont dénommés avec un signe (R) ou respectivement (S) à la suite immédiate de l'identification de la position d'un substituant, cela signifie que le carbone porteur de ce substituant est de configuration (R) ou respectivement (S), conformément aux règles de Cahn, Ingold et Prelog.

Les caractéristiques spectrales des signaux de résonance magnétique nucléaire (RMN) sont données pour le proton (¹H) ou pour l'isotope 13 du carbone (¹³C) : on indique le déplacement chimique par rapport au signal du tétraméthylsilane et, entre parenthèses, la forme du signal (s pour singulet, d pour doublet, t pour triplet, q pour quadruplet, m pour multiplet, sl pour signal large) et le nombre de protons concernés par le signal. A titre indicatif, les spectres RMN¹H ont été réalisés à 300 MHz.

### PREPARATION I

### Acide 3-[[(1,1-diméthyléthoxy)carbonyl]amino]-21-[(1,1-diméthyléthoxy)carbonyl]-12,15-dioxo-16-oxa-2,4,11,14,21,25-hexaazahexacos-2-énedioïque, bis(1,1-diméthyléthyl) ester.

On dissout 1 g (2,89.10⁻³ mole) d'acide [3-[[(1,1-diméthyléthoxy)carbonyl]amino]propyl] (4-hydroxybutyl)carbamique, 1,1-diméthyléthyl ester dans 20 ml de tétrahydrofuranne (THF), on ajoute 0,45 g (4,5.10⁻³ mole) de triéthylamine puis 0,58 g (2,89.10⁻³ mole) de chloroformiate de 4-nitrophényle en solution dans 5 ml de THF. Le mélange réactionnel est agité pendant 15 heures à température ambiante, puis on ajoute 1,2 g (2,89.10⁻³ mole) d'acide 13-amino-3-[[(1,1-diméthyléthoxy)carbonyl]amino]-12-oxo-2,4,11-triazatridéc-2-ènoïque, 1,1-diméthyléthyl ester en solution dans 6 ml de THF, on porte le mélange réactionnel à 40° C et on agite pendant 5 heures. Après concentration du milieu réactionnel sous pression réduite, le résidu est purifié par chromatographie à moyenne pression sur gel de silice en éluant avec un mélange méthylcyclohexane/acétate d'éthyle (7/3 ; v/v) puis avec de l'acétate d'éthyle pur. On obtient ainsi 1 g du produit attendu sous la forme d'une huile transparente (rendement = 44%).
RMN¹H (CDCl₃) : 1,3-1,9 (m,50H) ; 3,05-3,55 (m,10H) ; 3,85 (d, 2H) ; 4,10 (t,2H) ; 4,7-5,3 (sl,1H) ; 5,4-5,6 (sl,1H) ; 6,0-6,2 (sl,1H) ; 8,3-8,5 (sl,1H) ; 11,5 (s,1H).

### Exemple 1

### [4-[(3-Aminopropyl)amino]-butoxycarbonylamino]-N-[6-[(aminoiminométhyl)amino]hexyl]acétamide, tris(trifluoroacétate).

On prépare un mélange de 1 g (1,27.10⁻³ mole) du composé obtenu selon la préparation I, dans 10 ml de dichlorométhane et 10 ml d'acide trifluoroacétique et on agite le milieu réactionnel pendant 15 heures à température ambiante. Les solvants sont ensuite éliminés sous pression réduite et le résidu est purifié par chromatographie à moyenne pression (MPLC) sur silice greffée de type RP18 (granulométrie 5 à 20 µ m) en éluant avec un mélange éthanol/eau/acide trifluoroacétique (2/7,5/0,5 ; v/v). Les fractions contenant le produit pur sont concentrées sous pression réduite, reprises en solution dans de l'eau et lyophilisées. On obtient ainsi 0,78 g du produit attendu sous la forme d'un solide amorphe blanc translucide (rendement = 84 %).
RMN¹H (DMSO d₆) : 1,2-1,55 (m,8H) ; 1,6-1,75 (m,4H) ; 1,8-1,95 (m, 2H) ; 2,8-3,1 (m,10H) ; 3,55 (d,2H) ; 3,95 (t,2H) ; 6,7-7,4 (m,4H) ; 7,55 (t, 1H) ; 7,75-8,00 (m,4H) ; 8,5-8,65 (m,3H).
RMN¹³C (D₂O-dioxanne h₈) : 23,05 ; 24,55 ; 26,15 ; 26,20 ; 26,30 ; 28,55 ; 28,95 ; 31,05 ; 37,35 ; 39,95 ; 41,88 ; 45,24 ; 48,18 ; 65,71; 158,0 ; 159,0 ; 178,5.

### Exemple 1bis

### [4-[(3-Aminopropyl)amino]-butoxycarbonylamino]-N-[6-[(aminoiminométhyl)amino]hexyl]acétamide, tris(chlorhydrate).

On prépare une solution de 3,5 g du composé selon l'exemple 1, (4,8.10⁻³ mole) dans 7 ml d'éthanol anhydre et on ajoute à 0° C, goutte à goutte 10 ml d'une solution 1,3M de chlorure d'hydrogène dans l'éthanol. On maintient sous agitation pendant 30 minutes après la fin de l'addition puis on filtre le précipité obtenu. Après lavage du solide avec de l'éthanol anhydre, on sèche sous vide à 35-40° C. On obtient ainsi le produit attendu (1,72 g) sous la forme d'une poudre blanche hygroscopique (rendement = 72 %).
**F = 107,5° C**

### PREPARATION II

### Acide [3-[[4[tris(phényl)-méthoxy]butyl](phénylméthyl)-amino]-1-(R)méthylpropyl]-carbamique, 1,1-diméthyléthyl ester.

On prépare une solution de 3,77 g (13,5.10⁻³ mole) d'acide [3-(phénylméthylamino)-1-(R)-méthylpropyl]carbamique,1,1-diméthyléthyl ester dans 270 ml de butanol, puis on ajoute 12 g (27,1.10⁻³ mole) de 1-iodo-4-[tris(phényl)-méthoxy]butane et 3,74 g (27,1.10⁻³ mole) de carbonate de potassium. Le mélange réactionnel est agité à 95-100° C pendant 48 heures. Après refroidissement, le milieu réactionnel est filtré puis concentré sous pression réduite. Le résidu est ensuite repris avec du dichlorométhane et lavé à l'eau. Après concentration de la phase organique sous pression réduite, le produit est purifié par chromatographie sur gel de silice en éluant avec un mélange hexane/acétate d'éthyle (80/20 v/v). On obtient ainsi 6 g du produit attendu sous la forme d'une huile (rendement = 75%).
**[α]**^{**24**}_{**D**} **= - 0,22° (c = 1,8 ; CHCl**_{**3**}**).**
RMN¹H (CDCl₃) : 1,02 (d,3H) ; 1,40 (s,9H) ; 1,59 (m, 6H) ; 2,38 (m,3H) ; 2,55 (m,1H) ; 3,0 (sl,2H) ; 3,40 (d,1H) ; 3,6 (d,2H) ; 5,70 (s,1H) ; 7,21-7,43 (m,20H).

### PREPARATION III

### Acide [3-[[4-hydroxy-butyl]amino]-1-(R)-méthylpropyl]-carbamique, 1,1-diméthyléthyl ester (chlorhydrate).

On dissout 4,94 g (8,33.10⁻³ mole) du composé obtenu selon la préparation II dans 230 ml d'éthanol 95° [i.e. approximativement un mélange éthanol/eau (95/95 ; v/v)], puis on ajoute 4,37 ml d'une solution de HCl à 4,3 N dans l'éthanol à 95°, et 1 g de charbon palladié à 5 %. Le mélange est ensuite hydrogéné à pression atmosphérique pendant 40 heures, à température ambiante. Le catalyseur est éliminé par filtration et le filtrat est concentré sous pression réduite. Après purification par chromatographie sur gel de silice en éluant avec un mélange chloroforme/méthanol (9/1 ; v/v), on obtient 2,09 g du produit attendu. Après lavage avec de l'éther éthylique, on obtient 1,6 g de produit pur sous la forme d'un solide cristallin blanc (rendement = 64,8 %).
**F = 135° C**
**[α]**^{**22**}**D = - 7,6° (c= 1 (CHCl**_{**3**}**).**

### PREPARATION IV

### Acide [3-[(4-hydroxy-butyl)(1,1-diméthyléthoxycarbonyl)-amino]-1-(R)-méthylpropyl]-carbamique, 1,1-diméthyléthyl ester.

On prépare un mélange de 1,54 g (5,18.10⁻³ mole) du composé obtenu selon la préparation III, 8 ml de dioxanne et d'une solution de 0,77 g (7,26.10⁻³ mole) de carbonate de sodium dans 5,7 ml d'eau. On refroidit ce mélange à 5° C puis on ajoute une solution de 1,13 g (5,18.10⁻³ mole) de dicarbonate de di-tert-butyle (Boc₂O) dans 3 ml de dioxanne et on maintient le mélange réactionnel sous agitation pendant 15 heures. Après addition de 10 ml d'eau, on extrait avec de l'acétate d'éthyle, puis on concentre la phase organique sous pression réduite. Après purification par chromatographie sur gel de silice, en éluant avec un mélange hexane/acétate d'éthyle (50/50 ; v/v), on obtient 1,82 g du produit attendu sous la forme d'une huile visqueuse incolore (rendement = 97%).
**[α]**^{**22**}_{**D**} **= + 4,25° (c = 2 ; CHCl**_{**3**}**).**
RMN¹H (CDCl₃) : 0,87 (d,3H) ; 1,44-1,46 (2s,18H) ; 1,50-1,70 (m, 6H) ; 3,20 (m,4H) ; 3,67 (t,3H) ; 4,41-4,56 (2sl, 1H).

### PREPARATION V

### Acide [3-[N-[4-[(4-nitrophénoxy)carbonyloxy]-butyl]-N-[1,1-diméthyl-éthoxycarbonyl]-amino]-1-(R)-méthylpropyl]carbamique, 1,1-diméthyléthyl ester.

On prépare une solution de 2,73 g (7,57.10⁻³ mole) du composé obtenu selon la préparation IV dans 55 ml de toluène et 0,62 ml (7,7.10⁻³ mole) de pyridine et on ajoute 1,6 g (7,7.10⁻³ mole) de chloroformiate de 4-nitrophényle. On maintient le mélange réactionnel sous agitation pendant 3 heures puis on élimine les sels formés par filtration. Le filtrat est concentré sous pression réduite puis purifié par chromatographie sur gel de silice en éluant avec un mélange dichlorométhane/acétate d'éthyle (90/10 ; v/v). On obtient ainsi 3,1 g du produit attendu (rendement = 78%).
RMN¹H (CDCl₃) : 1,15 (d,3H) ; 1,44 (s,9H) ; 1,46 (s, 9H) ; 1,5-1,8 (m,6H) ; 3,24 (m,4H) ; 3,63 (m,1H) ; 4,30 (t,2H) ; 7,39 (d,2H) ; 8,28 (d,2H).

### PREPARATION VI

### Acide 3-[[(1,1-diméthyléthoxy)carbonyl]amino]-21-[(1,1-diméthyl éthoxy)carbonyl]-24-(R)-méthyl-12,15-dioxo-16-oxa-2,4,11,14,21,25-hexaaza-hexacos-2-ènedioïque, bis(1,1-diméthyléthyl) ester.

On dissout 2,8 g (5,33.10⁻³ mole) du composé selon la préparation V dans 300 ml de THF, puis on ajoute 0,85 ml (6,1.10⁻³ mole) de triéthylamine et une solution de 2,54 g (6,1.10⁻³ mole) d'acide 13-amino-3-[[(1,1-diméthyléthoxy)carbonyl]amino]-12-oxo-2,4, 11,-triazatridec-2-énoïque, 1,1-diméthyléthyl ester dans 50 ml de THF. On maintient sous agitation pendant 4 heures à température ambiante, puis on concentre le mélange réactionnel sous pression réduite. Le résidu solide est purifié par chromatographie sur gel de silice en éluant avec un mélange hexane/acétate d'éthyle (30/70, puis 10/90, v/v). On obtient ainsi 3,85 g du produit attendu sous la forme d'un solide jaune clair (rendement = 90,2 %).
**[α]**^{**20**}_{**D**} **= + 0,4° (c= 2 ; CHCl**_{**3**}**).**
RMN¹H (CDCl₃) : 1,15 (d,3H) ; 1,3-1,70 (m,50H) ; 3,25 (m, 6H) ; 3,39 (q,2H) ; 3,63 (m,1H) ; 3,83 (d,2H) ; 4,14 (t,2H) ; 4,6 (sl,1H) ; 5,60 (sl,1H) ; 6,13 (sl, 1H) ; 8,31 (s, 1H) ; 11,50 (s,1H).

### Exemple 2

### [4-[(3-(R)-Amino-butyl)amino]-butoxycarbonylamino]-N-[6-(amino iminométhylamino)hexyl]acétamide, tris(trifluoroacétate).

On refroidit à 0° C dans un ballon, 3,7 g (4,6.10⁻³ mole) du composé obtenu selon la préparation VI et on ajoute 37 ml d'acide trifluoroacétique. Le mélange est maintenu sous agitation pendant 15 heures à 5-10° C puis concentré sous pression réduite. Le produit brut est purifié par chromatographie sur gel de silice greffée de type RP18, en éluant avec un mélange eau/acétonitrile/acide trifluoroacétique (70/15/15; v/v). On obtient ainsi 3,1 g du produit attendu sous la forme d'un solide blanc hygroscopique (rendement = 92 %).
**[α]**^{**24**}_{**D**} **= + 0,89° (c = 2 ; CH**_{**3**}**OH).**
RMN¹H (DMSO d₆) : 1,18 (d,3H) ; 1,26 (m,4H) ; 1,41 (m, 4H) ; 1,61 (m,4H) ; 1,75 (m,1H) ; 1,91 (m,1H) ; 2,85-3,20 (m,8H) ; 3,28 (m, 1H) ; 3,54 (d,2H) ; 3,96 (sl,2H) ; 7,25 (t,1H) ; 7,64 (s, 1H) ; 7,86 (t, 1H) ; 7,98 (s,3H) ; 8,62 (s,2H).
RMN¹³C (D₂O + dioxanne) : 17,93 ; 22,98 ; 26,06 ; 26,14 ; 26,23 ; 28,49 ; 28,91 ; 31,13 ; 39,89 ; 41,79 ; 44,31 ; 44,52 ; 46,01; 48,08 ; 65,60 ; 157,44 ; 159,38 ; 172,64.

### Exemple 2 bis

### [4-[(3-(R)-Amino-butyl)amino]-butoxycarbonylamino]-N-[6-(amino iminométhylamino)hexyl]acétamide, tris(chlorhydrate).

On prépare une solution de 200 mg (0,27.10⁻³ mole) du composé obtenu à l'exemple 2 dans 0,5 ml d'éthanol et on ajoute 0,235 ml d'une solution 10,4 N de HCl dans l'éthanol. On agite 15 min à la température ambiante puis on ajoute 2 ml de (diisopropyl) éther. On sépare le produit précipité par décantation, on le rince avec du (diisopropyl) éther, puis on le reprend en solution dans 3 ml d'eau et on lyophilise. On obtient ainsi 117 mg du produit attendu sous la forme d'un solide blanc amorphe (rendement = 85 %).
**[α]**^{**22,5**}_{**D**} **= + 1,45 ° (c= 2; CH**_{**3**}**OH).**
RMN¹H (DMSO d₆) : 1,21 (d,3H) ; 1,26 (m,4H) ; 1,42 (m, 4H) ; 1,64 (m,4H) ; 1,88 (m,1H) ; 2,04 (m,1H) ; 2,89-3,10 (m,8H) ; 3,35-3,70 (m,3H) ; 3,97 (sl,2H) ; 7,32 (t,1H) ; 7,80 (t,1H) ; 7,92 (t,1H) ; 8,24 (s,3H) ; 9,16 (s,2H).

### PREPARATION VII

### Acide [3-[N-[4-hydroxy-butyl]amino]-1-(R,S)-méthylpropyl]-carbamique, 1,1-diméthyléthyl ester.

On prépare une solution de 6,2 g (23,25.10⁻³ mole) d'acide [1-(R, S)-méthyl-3- [(méthylsulfonyl)oxy]propyl]carbamique, 1,1 -diméthyléthyl ester, dans 40 ml de 1,2-diméthoxyéthane et on ajoute goutte à goutte une solution de 4,14 g (46,5.10⁻³ mole) de 4-aminobutanol dans 10 ml de 1,2-diméthoxyéthane. Le mélange réactionnel est porté au reflux pendant 18 heures puis concentré sous pression réduite. Le produit brut est purifié par chromatographie sur gel de silice en éluant avec un mélange acétate d'éthyle/éthanol/ammoniaque 33 % (6/3/0,1 ; v,v). on obtient ainsi 2,5 g du produit attendu sous la forme d'une huile (rendement = 41,5 %).
RMN¹H (CDCl₃) : 1,14 (d,3H) ; 1,44 (s,9H) ; 1,5-1,8 (m, 6H) ; 2,64 (m,4H) ; 3,58 (t,2H) ; 3,71 (m,1H) ; 4,50 (d, 1H).

### PREPARATION VIII

### Acide [3-[N-(4-hydroxy-butyl)-N-(1,1-diméthyléthoxycarbonyl)-amino] -1-(R,S)-méthyl-propyl]-carbamique, 1,1-diméthyléthyl ester.

En opérant de façon analogue au procédé de la préparation IV, au départ du composé obtenu selon la préparation VII, on obtient le produit attendu avec un rendement de 70 %.
RMN¹H (CDCl₃) : 1,15 (d,3H) ; 1,44-1,46 (2s,18H) ; 1,5-1,7 (m, 6H) ; 3,20 (m,4H) ; 3,67 (t,3H) ; 4,39-4,56 (2sl, 1H).

### PREPARATION IX

### Acide 3-[[(1,1-diméthyléthoxy)carbonyl]amino]-21-[(1,1-diméthyléthoxy)carbonyl]-24-(R,S)-méthyl-12,15-dioxo-16-oxa-2,4,11,14,21,25-hexaaza-hexacos-2-énedidique, bis(1,1-diméthyléthyl) ester.

On dissout 1,8 g (5.10⁻³ mole) du composé obtenu selon la préparation VIII ci-dessus, dans 36 ml de THF, puis on ajoute 1,1 g (7,8.10⁻³ mole) de triéthylamine et 1,10 g (5,45.10⁻³ mole) de chloroformiate de 4-nitrophényle. Le milieu réactionnel est maintenu sous agitation pendant 15 heures puis on ajoute une solution de 2,1 g (5,06.10⁻³ mole) d'acide 13-amino-3-[[(1,1-diméthyléthoxy)carbonyl]amino]-12-oxo-2,4,11-triaza-tridec-2-énoïque, 1,1-diméthyléthyl ester, dans 30 ml de THF et on maintient sous agitation pendant 18 heures. Le solide formé par la réaction est éliminé par filtration puis la solution est concentrée sous pression réduite et le résidu est purifié par chromatographie sur gel de silice en éluant avec un mélange méthylcyclohexane/acétate d'éthyle (50/50) puis (20/80 ; v/v). On obtient ainsi 0,97 g du produit attendu sous la forme d'une huile (rendement = 24,2 %).
RMN¹H (CDCl₃) : 1,15 (d,3H) ; 1,20-1,80 (m,50H) ; 3,26 (m, 6H) ; 3,39 (q,2H) ; 3,62 (m,1H) ; 3,83 (d,2H) ; 4,12 (m,2H) ; 4,60 (sl,1H) ; 5,60 (sl, 1H) ; 6,13 (sl,1H) ; 8,30 (t, 1H) ; 11,49 (s, 1H).

### Exemple 3

### [4-[(3-(R,S)-Amino-butyl)amino]-butoxycarbonylamino]-N-[6-(amino iminométhylamino)hexyl]acétamide, tris(trifluoroacétate).

On dissout 0,95 g (1,18.10⁻³ mole) du composé obtenu selon la préparation IX dans 10 ml de dichlorométhane puis on ajoute 10 ml d'acide trifluoroacétique. Après 24 heures sous agitation à température ambiante, le mélange réactionnel est concentré sous pression réduite et le résidu est purifié par chromatographie sur gel de silice greffée de type RP18, en éluant avec un mélange acétonitrile/eau/acide trifluoroacétique (15/80/5 ; v/v). Après lyophilisation des fractions pures, on obtient 580 mg du produit attendu sous la forme d'un solide blanc (rendement = 66 %).
RMN¹H (DMSO d₆) : 1,18 (d,3H) ; 1,26 (m,4H) ; 1,41 (m, 4H) ; 1,61 (m,4H) ; 1,74 (m,1H) ; 1,91 (m,1H) ; 2,85-3,10 (m,8H) ; 3,28 (m,1H) ; 3,54 (d,2H) ; 3,96 (t,2H) ; 7,23 (t,1H) ; 7,58 (t,1H) ; 7,84 (t,1H) ; 7,93 (s,3H) ; 8,56 (m,2H).
RMN¹³C (D₂O + dioxanne h₈) : 18,02 ; 23,09 ; 26,17 ; 26,22 ; 26,30 ; 28,58 ; 29,00 ; 31,24 ; 40,00 ; 41,88 ; 44,40 ; 44,63 ; 46,10 ; 48,19 ; 65,72 ; 157,55 ; 159,55 ; 172,82.

### PREPARATION X

### N-(6-Cyanohexanoyl)glycine, éthyl ester.

On dissout 23,73 g (84,7.10⁻³ mole) de N-(6-bromohexanoyl)glycine, éthyl ester, dans 200 ml d'éthanol et on ajoute 6,5 g (0,1 mole) de cyanure de potassium en poudre. Le mélange réactionnel est porté au reflux et agité ainsi pendant 15 heures. Après concentration du mélange sous pression réduite le résidu est repris par du dichlorométhane et la phase organique lavée avec une solution aqueuse de chlorure de sodium. La phase organique est séchée, et concentrée sous pression réduite. On obtient ainsi 19 g du produit attendu (rendement = 99 %).
RMN¹H (CDCl₃) : 1,29 (t,3H) ; 1,45-1,55 (m,2H) ; 1,6-1,8 (m, 4H) ; 1,27 (t,2H) ; 2,36 (t,2H) ; 4,03 (d,2H) ; 4,22 (q,2H) ; 5,35-6,05 (sl,1H).

### PREPARATION XI

### N-(6-Cyanohexanoyl)glycine.

On prépare un mélange de 19 g (84.10⁻³ mole) du composé obtenu selon la préparation X, 120 ml de 1,2-diméthoxyéthane et 120 ml d'une solution de soude 1M et on agite pendant 15 min à température ambiante. On ajoute ensuite 100 ml d'eau et 200 ml de dichlorométhane, et on acidifie jusqu'à pH=1 en refroidissant au bain de glace. La phase aqueuse est extraite plusieurs fois au dichlorométhane et les phases organiques rassemblées sont séchées et concentrées sous pression réduite. On obtient ainsi 10,2 g du produit attendu sous la forme d'huile jaune paille (rendement = 61 %).
RMN¹H (CDCl₃) : 1,45-1,55 (m,2H) ; 1,6-1,8 (m, 4H) ; 2,3 (t,2H) ; 2,36 (t,2H) ; 4,08 (d,2H) ; 6,15-6,25 (sl, 1H).

### PREPARATION XII

### N-(7-Amino-heptanoyl)glycine (sel de sodium).

On dissout 8,2 g (41,4.10⁻³ mole) de N-(6-cyanohexanoyl)glycine dans 100 ml d'éthanol, on ajoute 60 ml d'une solution de soude 1M et 800 mg de nickel de Raney. Le mélange est ensuite agité sous atmosphère d'hydrogène, à température ambiante et sous une pression de 3,5.10⁵ Pa, pendant 8 heures. Après la fin de la réaction, on ajoute 20 ml d'acide chlorhydrique 1M et on concentre sous pression réduite ; on obtient ainsi 10,2 g d'un solide pâteux blanc qui contient le sel attendu et du chlorure de sodium et qui peut être utilisé sans autre purification à l'étape suivante.
RMN¹H (DMSO d₆) : 1,15-1,6 (m,8H) ; 2,08 (t, 2H) ; 2,56 (t,2H) ; 3,32 (d,2H) ; 7,1-7,25 (sl, 1H).

### PREPARATION XIII

### N-[7[(Phénylméthoxycarbonyl)amino]heptanoyl]glycine.

On dissout 2 g du composé obtenu selon la préparation XII dans un mélange de 50 ml d'eau et 50 ml d'éthanol, on ajoute 1,06 g (10⁻² mole) de carbonate de sodium puis 2,34 g (19,8.10⁻³ mole) de chloroformiate de benzyle. Après agitation pendant 15 heures à température ambiante, le milieu réactionnel est amené à pH=1 avec de l'acide chlorhydrique 1M, puis extrait avec du dichlorométhane. La phase organique est séchée et concentrée sous pression réduite et le produit brut est purifié par chromatographie sur gel de silice en éluant avec un mélange acétate d'éthyle/éthanol/ammoniaque (6/3/0,5, v/v). On obtient ainsi 1,3 g du produit attendu sous la forme d'un solide pâteux.
RMN¹H (DMSO d₆) : 1,2-1,6 (m,8H) ; 2,09 (t, 2H) ; 2,98 (td,2H) ; 3,64 (d,2H) ; 5,01 (s,2H) ; 7,25 (t, 1H) ; 7,3-7,4 (m,5H) ; 7,94 (t, 1H).

### PREPARATION XIV

### Acide 19-[(1,1-diméthyléthoxy)carbonyl]-9,13-dioxo-14-oxa-2,10,12,19,23-pentaaza-tetracosanedioïque, 1-(phénylméthyl) 24-(1,1-diméthyléthyl) ester.

On dissout 0,5 g (1,49.10⁻³ mole) de l'acide obtenu selon la préparation XIII dans 25 ml de THF, on ajoute 0,18 g (1,8.10⁻³ mole) de triéthylamine et on refroidit le mélange à 0° C; On ajoute ensuite goutte à goutte 0,46 g (1,67.10⁻³ mole) d'azoture de diphénylphosphoryle en solution dans 5 ml de THF et on laisse sous agitation à température ambiante pendant 45 minutes. On élimine le solvant sous pression réduite, on reprend le résidu dans 2 ml de toluène et on ajoute 0,18 g (1,8.10⁻³ mole) de triéthylamine et 1,04 g (3.10⁻³ mole) d'acide [3-[[(1,1-diméthyléthoxy)carbonyl]amino]propyl] (4-hydroxybutyl)carbamique, 1,1-diméthyléthyl ester. Le mélange réactionnel est porté à reflux pendant 20 heures puis concentré sous pression réduite. Le produit brut est purifié par chromatographie sur gel de silice en éluant avec un mélange méthylcyclohexane/acétate d'éthyle en gradient de (8/2 à 1/9 ; v/v). On obtient ainsi 0,4 g du produit attendu sous la forme d'un solide blanc (rendement = 39,6 %).
**F = 115° C**
RMN¹H (CDCl₃) : 1,2-1,7 (m,32H) ; 2,15 (t,2H) ; 3,05-3,3 (m, 8H) ; 4,05 (t,2H) ; 4,5 (t,2H) ; 4,7-4,9 (sl,1H) ; 5,08 (s,2H) ; 5,7-5,9 (sl,1H) ; 6,4-6,6 (sl, 1H) ; 7,3-7,4 (m,6H).

### PREPARATION XV

### Acide 22-amino-6-[(1,1-diméthyléthoxy)carbonyl]-12,16-dioxo-11-oxa-2,6,13,15-tetraaza-docosanoïque, 1,1-diméthyléthyl ester.

On soumet une solution de 0,33 g (0,48.10⁻³ mole) du composé obtenu selon la préparation XIV dans 15 ml d'éthanol à une hydrogénation catalytique en présence de 30 mg de charbon palladié à 5 %, à pression atmosphérique et à température ambiante pendant 5 heures. Après élimination du catalyseur par filtration, le solvant est chassé sous pression réduite et on obtient 0,26 g du produit attendu sous la forme d'une huile (rendement = 99 %).
RMN¹H (CDCl₃) : 1,2-1,75 (m,32H) ; 2,2 (t,2H) ; 2,8 (t, 2H) ; 2,85-3,3 (m,8H) ; 4,07 (t,2H) ; 4,53 (t,2H) ; 4,7-5,4 (sl,1H) ; 5,7-6,1 (sl,1H) ; 6,4-7,1 (sl,1H).

### PREPARATION XVI

### Acide 3-[[(1,1-diméthyléthoxy)carbonyl]amino]-21-[(1,1-diméthyl éthoxy)carbonyl]-11,15-dioxo-16-oxa-2,4,12,14,21,25-hexaaza-hexacos-2-énedioïque, bis(1,1-diméthyléthyl) ester.

On prépare un mélange de 0,26 g (0,477.10⁻³ mole) du composé obtenu selon la préparation XV, 0,276 g (0,95.10⁻³ mole) d'acide [[[(1,1-diméthyléthoxy)carbonyl]amino](méthylthio)méthylène]carbamique, 1,1-diméthyléthyl ester et 200 µl de triethylamine dans 12 ml de THF et on maintient le milieu réactionnel sous agitation pendant 4 jours, à température ambiante. Après évaporation du solvant sous pression réduite, le produit brut est purifié par chromatographie sur gel de silice en éluant avec un mélange méthylcyclohexane/acétate d'éthyle en gradient de (7/3 à 2/8 ; v/v). On obtient ainsi 0,10 g du produit attendu sous la forme d'une huile (rendement = 26,7 %).
RMN¹H (CDCl₃) : 1,0-1,8 (m,50H) ; 2,16 (t,2H) ; 3,05-3,3 (m, 6H) ; 3,4 (td,2H) ; 4,07 (t,2H) ; 4,55 (t,2H) ; 5,2-5,3 (sl,1H) ; 5,7-5,8 (sl,1H) ; 6,45-6,55 (sl, 1H) ; 8,3 (t, 1H) ; 11,5 (s,1H).

### Exemple 4

### 7-[(Aminoiminométhyl)amino]-N-[[4-[(3-aminopropyl)amino]butoxy] carbonylaminométhyl]heptanamide, tris(trifluoroacétate).

En opérant, au départ du composé obtenu selon la préparation XVI, de façon analogue au procédé de l'exemple 3, on obtient le produit attendu avec un rendement de 54 %.
RMN¹H (DMSO d₆) : 1,2-1,35 (m,4H) ; 1,4-1,7 (m,8H) ; 1,8-1,95 (m, 2H) ; 2,06 (t,2H) ; 2,8-3,1 (m,8H) ; 3,96 (t,2H) ; 4,32 (t,2H) ; 6,85-7,4 (sl,3H) ; 7,55-7,7 (m,2H) ; 7,8-8,0 (m,4H) ; 8,33 (t, 1H) ; 8,55-8,7 (m,2H).

### PREPARATION XVII

### Acide 19-[(1,1-diméthyléthoxy)carbonyl]-22-(R)-méthyl-9,13-dioxo-14-oxa-2,10,12,19,23-pentaaza-tetracosanedioïque, 1-(phénylméthyl) 24-(1,1-diméthyléthyl) ester.

En opérant de façon analogue au procédé de la préparation XIV, au départ d'acide obtenu selon la préparation XIII et d'alcool obtenu selon la préparation IV, on obtient le produit attendu sous la forme d'un solide amorphe avec un rendement de 46 %.
**[α]**^{**24**}_{**D**} **= - 2,2° (c= 1,06 ; CHCl**_{**3**}**).**
RMN¹H (CDCl₃) : 1,15 (d,3H) ; 1,2-1,7 (m,32H) ; 2,15 (t, 2H) ; 3,05-3,35 (m,6H) ; 3,55-3,7 (m,1H) ; 4,05 (t,2H) ; 4,55 (t,2H) ; 4,8-4,9 (sl,1H) ; 5,09 (s,2H) ; 5,1-5,2 (sl,1H) ; 5,7-5,9 (sl,1H) ; 6,45-6,6 (sl, 1H) ; 7,3-7,4 (m,5H).

### PREPARATION XVIII

### Acide 22-amino-6-[(1,1-diméthyléthoxy)carbonyl]-3-(R)-méthyl-12,16-dioxo-11-oxa-2,6,13,15-tétraaza-docosanoïque, 1,1-diméthyléthyl ester.

En opérant de façon analogue au procédé de la préparation XV, au départ du composé obtenu selon la préparation XVII, on obtient le produit attendu sous forme d'huile avec un rendement de 99 %.
**[α]**^{**20**}_{**D**} **= - 2,1° (c= 1 ; CHCl**_{**3**}**).**
RMN¹H (CDCl₃) : 1,15 (d, 3H) ; 1,2-1,7 (m, 32H) ; 2,19 (t, 2H) ; 2,77 (t,2H) ; 2,85-3,3 (m,6H) ; 3,55-3,7 (m,1H) ; 4,07 (t,2H) ; 4,52 (t,2H) ; 5,7-6,3 (sl,2H) ; 6,8-7,0 (sl,1H).

### PREPARATION XIX

### Acide 22-[(aminoiminométhyl)amino]-6-[(1,1-diméthyléthoxy)carbonyl]-3(R)-méthyl-12,16-dioxo-11-oxa-2,6,13,15-tétraaza-docosanoïque, 1,1-diméthyléthyl ester.

On dissout 0,4 g (0,716.10⁻³ mole) du composé obtenu selon la préparation XVIII dans 10 ml de méthanol, on ajoute 0,186 g (1,5.10⁻³ mole) d'acide aminoiminométhanesulfonique et on agite le mélange réactionnel à température ambiante pendant 3 jours. Après élimination du solvant sous pression réduite, le produit brut est purifié par chromatographie sur gel de silice en éluant avec un mélange acétate d'éthyle/éthanol/ammoniaque (6/3/0,1 puis 6/3/1 ; v/v). On obtient ainsi 0,37 g du produit attendu sous la forme d'une huile (rendement = 86 %).
**[α]**^{**22**}_{**D**} **= -1° (c= 0,89 ; CHCl**_{**3**}**).**
RMN¹H (CDCl₃) : 1,15 (d,3H) ; 1,2-1,7 (m,32H) ; 2,2 (t, 2H) ; 2,9-3,3 (m,6H) ; 3,5-3,75 (m,1H) ; 4,05 (t,2H) ; 4,52 (t,2H) ; 5,4-5,7 (sl,2H) ; 6,3-6,5 (sl,1H) ; 7,05-7,3 (sl,4H) ; 7,8-8,0 (sl,1H).

### Exemple 5

### 7-[(Aminominométhyl)amino]-N-[[4-[(3(R)-aminobutyl)amino] butoxy]carbonylaminométhyl]heptanamide, tris(trifluoroacétate).

En opérant de façon analogue au procédé de l'exemple 3, au départ du composé obtenu selon la préparation XIX, on obtient le produit attendu sous la forme d'un solide amorphe, avec un rendement de 42%.
**[α]**^{**23**}_{**D**} **= + 1,2° (c= 1 ; CH**_{**3**}**OH).**
RMN¹H (DMSO d₆) : 1,18 (d,3H) ; 1,2-1,35 (m,4H) ; 1,4-1,55 (m, 4H) ; 1,55-1,7 (m,4H) ; 1,7-1,85 (m,1H) ; 1,85-2,0 (m,1H) ; 2,05 (t,2H) ; 2,85-3,1 (m,6H) ; 3,2-3,35 (m,1H) ; 3,96 (t,2H) ; 4,32 (t,2H) ; 6,8-7,5 (sl,3H) ; 7,60 (t,1H) ; 7,68 (t,1H) ; 7,95 (s,4H) ; 8,35 (t, 1H) ; 8,5-8,7 (m,2H).
RMN¹³C (D₂O + dioxanne h₈) : 18,0 ; 23,06 ; 25,79 ; 26,12 ; 26,25 ; 28,45 ; 28,50 ; 31,22 ; 36,27 ; 41,86 ; 44,61 ; 46,08 ; 46,55 ; 48,17 ; 65,48 ; 157,52 ; 159,06 ; 178,36.

L'activité immunosuppressive des produits selon l'invention a été mise en évidence à l'aide d'un test dit de réaction du greffon contre l'hôte. Des souris mâles B6D2F1 (hybrides de première génération C57B1/6 x DBA/2) sont immunodéprimées par une injection intrapéritonéale (i.p) de cyclophosphamide. Trois jours plus tard (jour 0 de l'expérience : J₀), elles reçoivent par voie intraveineuse 4 x 10⁷ splénocytes de souris C57B1/6. Les animaux sont ensuite répartis par lot de 8 au minimum et reçoivent un traitement journalier de J₁ à J₅ et de J₇ à J₁₀ par voie i.p. Le groupe contrôle reçoit le véhicule seul. La mortalité est suivie jusqu'à J₆₀. Les résultats, exprimés par la valeur moyenne de la survie en jours à la dose indiquée, sont regroupés dans le tableau I où les valeurs données sont significatives selon le test de Logrank (probabilité inférieure ou égale à 5%). Pour comparaison, on a également indiqué dans le tableau I les valeurs obtenues avec des produits connus (ou de structure apparentée) de l'art antérieur : (A) la 15-déoxyspergualine [sous forme de tris(chlorhydrate)] et le composé (B) qui correspond à l'exemple 16 de EP-A-0 600 762.

### Produit B :

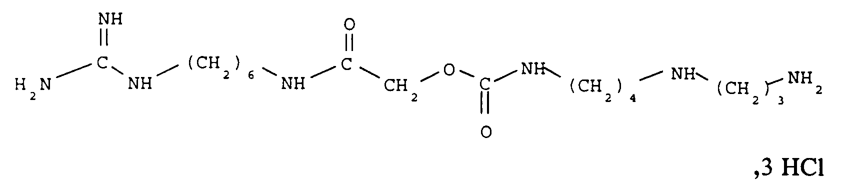

Il ressort de cette comparaison que les produits selon l'invention présentent une meilleure activité que les produits de l'art antérieur, ou nécessitent une plus faible posologie pour obtenir une activité équivalente.
Les produits selon l'invention sont utiles en thérapeutique en tant qu'agents immunosuppresseurs curatifs ou préventifs, notamment dans la prévention du rejet d'organes allogéniques ou xénogéniques vasculaires ou non, de la réaction du greffon contre l'hôte suite à une greffe vascularisée ou non, dans le traitement des maladies auto-immunes génétiquement définies ou acquises (comme par exemple le lupus érythémateux disséminé, la sclérose en plaques, la polyarthrite rhumatoÍde), des maladies inflammatoires chroniques telles que par exemple les rhumatismes articulaires ainsi que dans toutes les pathologies où un désordre immunitaire apparaît être la cause ou le facteur responsable du maintien d'un état clinique dégradé.

Les produits selon l'invention peuvent également être administrés en complément de drogues anticancéreuses cytotoxiques afin d'en limiter les effets secondaires et en complément de l'administration de produits issus des biotechnologies, notamment les cytokines recombinantes, les anticorps mono- et polyclonaux afin de diminuer l'apparition des anticorps protecteurs produits par le patient.

Les produits selon l'invention peuvent être utilisés dans le traitement curatif de parasitoses, en particulier dans le cas du paludisme.

Les produits selon l'invention peuvent être administrés par voie orale, par voie injectable (notamment par voie intramusculaire ou intraveineuse), par voie topique (notamment sous forme de crème pour application locale, de gouttes oculaires), par voie transdermique, par voie rectale sous forme de suppositoire, ou par inhalation.
Les produits selon l'invention trouvent également leur utilité en tant que réactifs pharmacologiques, notamment dans l'étude des maladies auto-immunes.

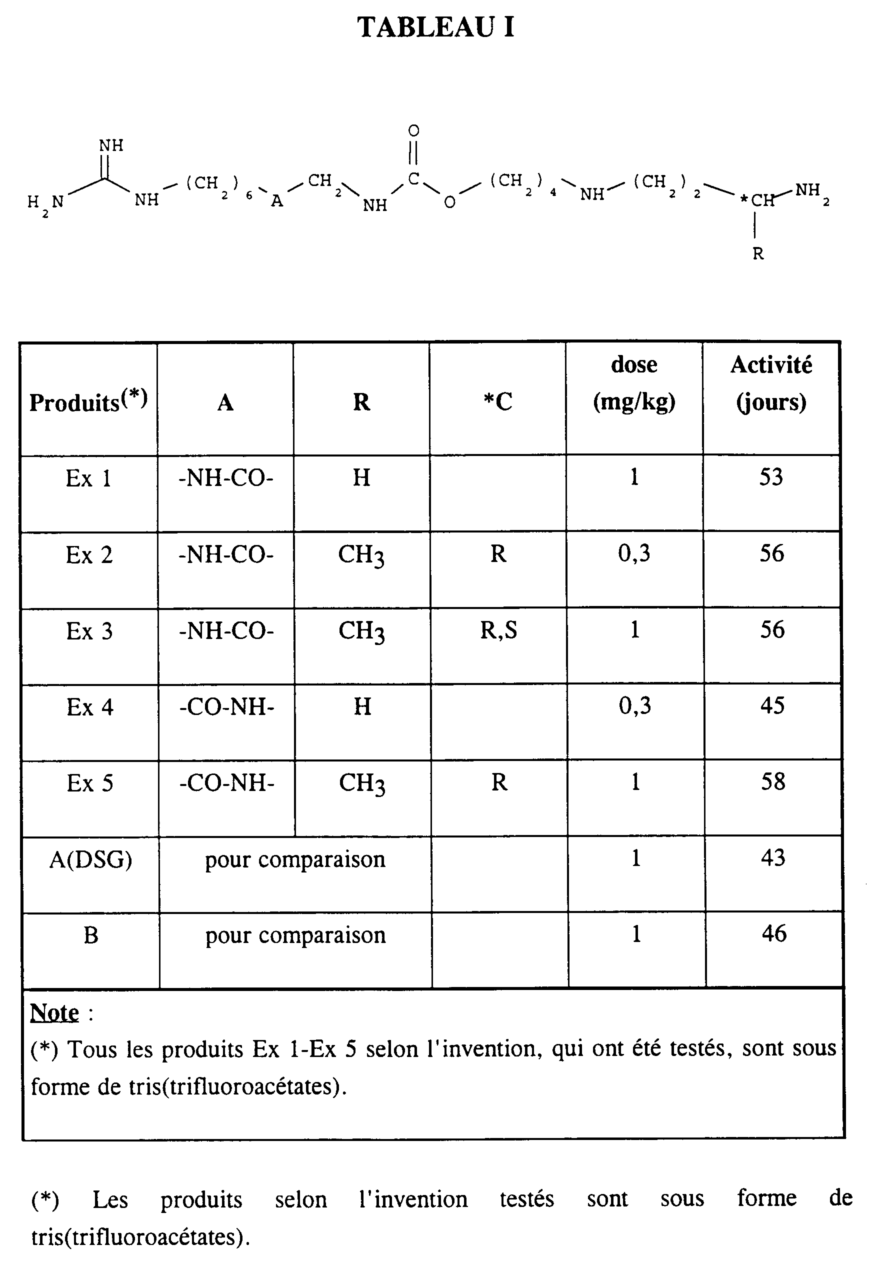

## Revendications

1. Composé de la famille des analogues de la 15-déoxy-spergualine, caractérisé en ce qu'il est choisi parmi l'ensemble constitué par :
**(i)** les composés de formule : 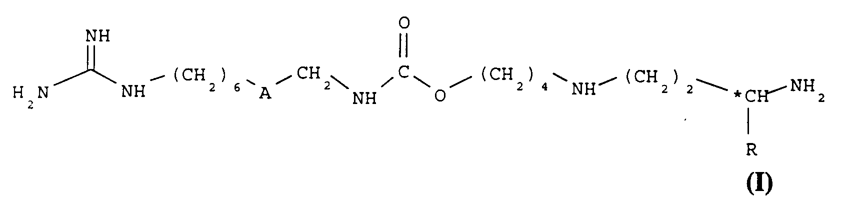 dans laquelle :
A représente un groupe -CO-NH- ou un groupe -NH-CO-,
R représente un atome d'hydrogène ou un groupe CH₃,
*C représente, lorsque R n'est pas l'atome d'hydrogène, un carbone asymétrique de configuration (R,S) ou de configuration (R),
et
**(ii)** leurs sels d'addition.

2. Composé selon la revendication 1, caractérisé en ce que R représente un groupe méthyle et le carbone asymétrique *C est de configuration (R).

3. Procédé de préparation d'un composé de formule I ou de l'un de ses sels d'addition selon la revendication 1, ledit procédé étant caractérisé en ce qu'il comprend les étapes consistant à:
**(i)** déprotéger un composé de formule : 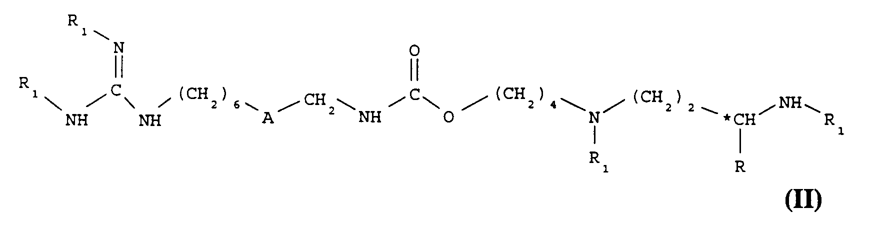 dans laquelle :
R représente un atome d'hydrogène ou un groupe méthyle,
A représente un groupe -CO-NH- ou un groupe -NH-CO-,
*C représente, lorsque R n'est pas l'atome d'hydrogène, un carbone asymétrique de configuration (R,S) ou (R), l'un au moins des substituants R₁ représente un groupe amino-protecteur de type oxycarbonyle ou benzyle, et les autres substituants R₁, s'il y a lieu, représentent un atome d'hydrogène,
selon un traitement adapté à la nature du groupe amino-protecteur, pour obtenir un composé de formule I sous forme de base libre ou de l'un de ses sels d'addition et, si nécessaire,
**(ii)** à partir de la base libre ou de son sel d'addition obtenu selon l'étape (i), obtenir les autres sels d'addition.

4. Procédé selon la revendication 3, caractérisé en ce qu'il comprend en outre les étapes choisies parmi l'ensemble constitué par :
**(a)** la variante A, qui comprend les étapes consistant à :
**(i)** condenser un alcool de formule : 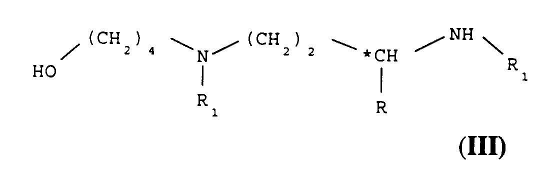 dans laquelle :
R représente un atome d'hydrogène ou un groupe méthyle,
R₁ représente un groupe amino-protecteur,
*C représente, lorsque R n'est pas l'atome d'hydrogène, un carbone asymétrique de configuration (R,S) ou (R),
avec un chloroformiate ou un carbonate symétrique en présence d'une base, dans un solvant inerte, à la température ambiante (15-25° C),
**(ii)** faire agir sur le composé obtenu au stade (i), une amine de formule : 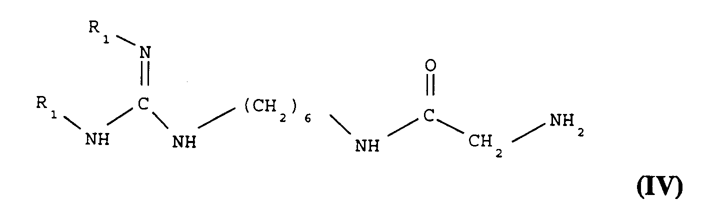 dans laquelle R₁ représente un groupe amino-protecteur, dans un solvant inerte, à une température d'environ 25 à 50° C, pour obtenir un composé de formule : 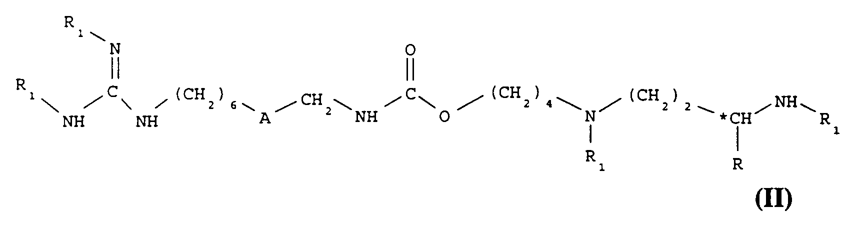 dans laquelle :
A représente le groupe -NH-CO-,
R représente un atome d'hydrogène ou un groupe méthyle,
*C représente, lorsque R n'est pas l'atome d'hydrogène, un carbone asymétrique de configuration (R,S) ou (R),
R₁ représente un groupe amino-protecteur;
**(b)** la variante B qui comprend les étapes consistant à :
**(i)** faire réagir un acide de formule : 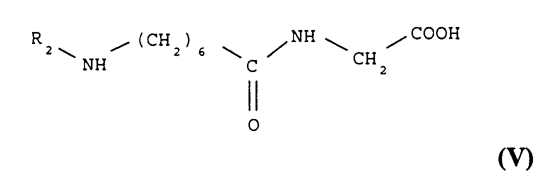 dans laquelle R₂ représente un groupe amino-protecteur, avec de l'azoture de diphénylphosphoryle de formule : 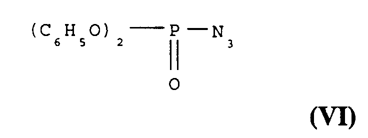 en présence d'une base, dans un solvant, à température ambiante pour obtenir un composé intermédiaire de formule : 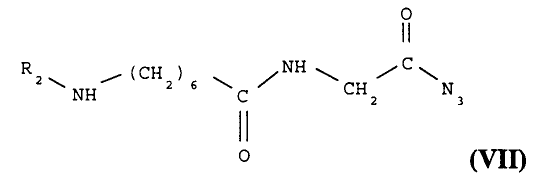
**(ii)** faire subir au composé VII obtenu ci-dessus un réarrangement connu sous le nom de réaction de Curtius et simultanément faire réagir l' isocyanate ainsi obtenu avec un alcool de formule : 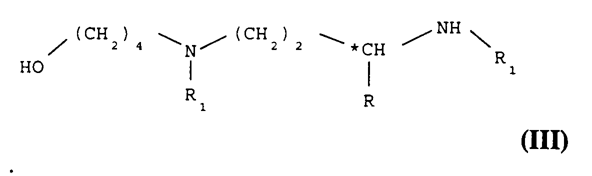 dans laquelle :
R représente un atome d'hydrogène ou un groupe méthyle,
R₁ représente un groupe amino-protecteur différent du groupe R₂ ci-dessus,
*C représente, lorsque R n'est pas l'atome d'hydrogène, un carbone asymétrique de configuration (R,S) ou (R),
dans un solvant, à une température d'environ 80 à 140° C, pendant 5 à 50 heures pour obtenir un composé de formule : 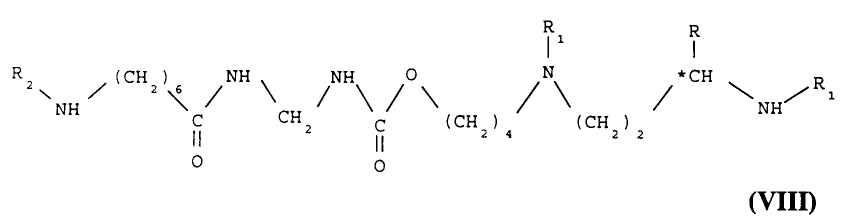 dans laquelle R, R₁, R₂ et *C ont les mêmes significations que ci-dessus,
**(iii)** déprotéger le composé VIII obtenu ci-dessus, selon un traitement spécifique au remplacement du groupe amino-protecteur R₂ par un atome d'hydrogène, pour obtenir un composé de formule : 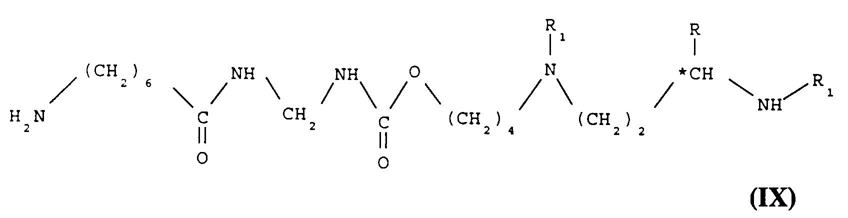 dans laquelle R, R₁ et *C ont les mêmes significations que ci-dessus,
**(iv)** faire réagir le composé de formule IX obtenu au stade (iii) avec l'acide amino-iminométhanesulfonique, dans un solvant, à température ambiante (15 à 25° C) et pendant 8 à 50 heures, pour obtenir un composé de formule : 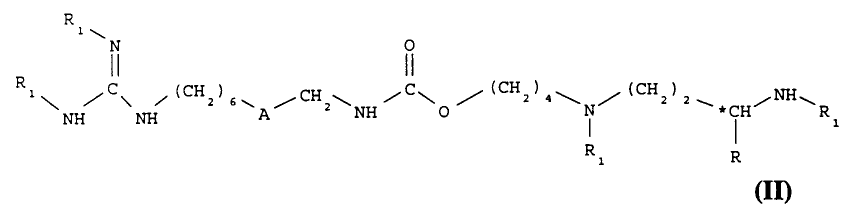 dans laquelle :
R et *C ont la même signification que ci-dessus,
A représente le groupe -CO-NH-,
R₁ représente un groupe amino-protecteur, exception faite des deux groupes R₁ portés par la fonction guanidine qui représentent chacun un atome d'hydrogène ; ou,
**(iv')** en variante de l'étape (iv) ci-dessus, on fait réagir le composé de formule IX avec un composé de formule : 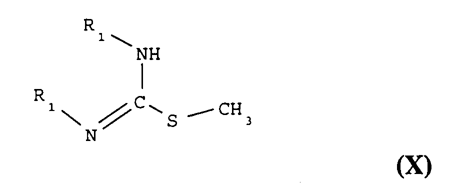 dans laquelle :
R₁ représente un groupe amino-protecteur,
dans un solvant inerte, notamment le tetrahydrofuranne, en présence d'une base, à température ambiante (15-25° C) pendant 8 à 100 heures pour obtenir un composé de formule : 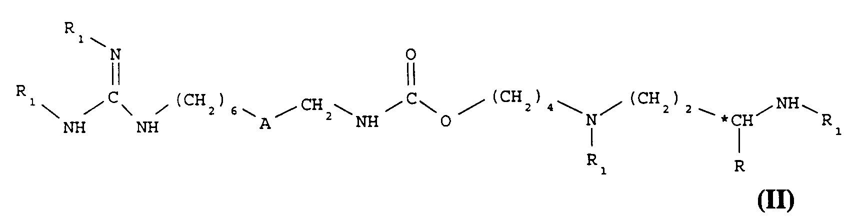 dans laquelle :
A représente le groupe -CO-NH-,
R représente un atome d'hydrogène ou un groupe méthyle,
R₁ représente un groupe amino-protecteur,
*C représente, lorsque R n'est pas l'atome d'hydrogène, un carbone asymétrique de configuration (R,S) ou (R).

5. Composé intermédiaire, utile dans la préparation d'un composé de formule I selon la revendication 1, caractérisé en ce qu'il est choisi parmi l'ensemble constitué par les composés de formule : 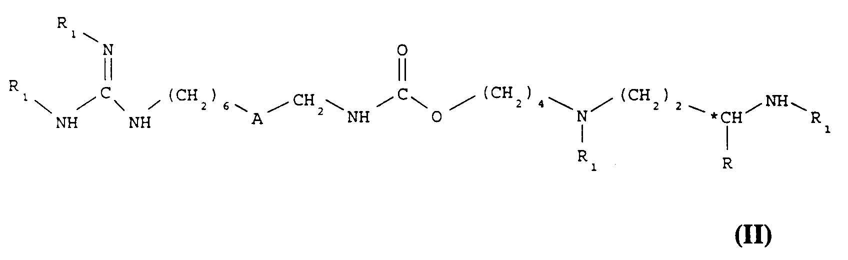 dans laquelle :
R représente un atome d'hydrogène ou un groupe méthyle,
A représente un groupe -CO-NH- ou un groupe -NH-CO-,
*C représente, lorsque R n'est pas l'atome d'hydrogène, un carbone asymétrique de configuration (R,S) ou de configuration (R),
l'un au moins des substituants R₁ représente un groupe amino-protecteur de type oxycarbonyle ou benzyle, et les autres substituants R₁, s'il y a lieu, représentent un atome d'hydrogène.

6. Composé intermédiaire, utile dans la préparation d'un composé de formule I selon la revendication 1, caractérisé en ce qu'il répond à la formule : 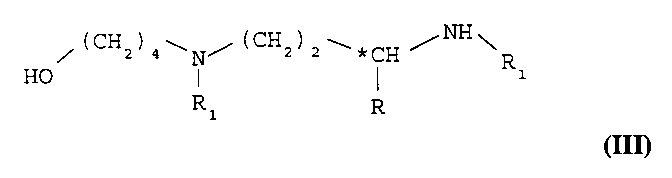 dans laquelle
R représente un groupe méthyle,
R₁ représente un groupe amino-protecteur,
*C représente un carbone asymétrique de configuration (R,S) ou (R).

7. Utilisation d'une substance immunosuppressive, choisie parmi l'ensemble constitué par les composés de formule I et leurs sels d'addition non-toxiques selon la revendication 1, pour l'obtention d'un médicament destiné à une utilisation en thérapeutique vis-à-vis des désordres immunitaires.

8. Utilisation d'une substance choisie parmi l'ensemble constitué par les composés de formule I et leurs sels d'addition non-toxiques selon la revendication 1, pour l'obtention d'un médicament destiné à une utilisation en thérapeutique vis-à-vis du paludisme.

9. Utilisation d'une substance choisie parmi l'ensemble constitué par les composés de formule I et leurs sels d'addition selon la revendication 1, en tant que réactif pharmacologique.

10. Composition thérapeutique caractérisée en ce qu'elle renferme, en association avec un excipient physiologiquement acceptable, au moins un composé choisi parmi l'ensemble constitué par les composés de formule I et leurs sels d'addition non-toxiques selon la revendication 1.

## Claims

1. A compound of the family of the 15-deoxyspergualin analogs which is selected from the group consisting of:
**(i)** the compounds of the formula 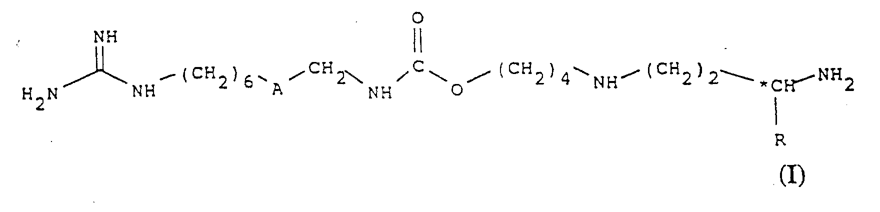 in which:
A is a group -CO-NH- or a group -NH-CO-,
R is a hydrogen atom or a group CH₃, and
*C, if R is not the hydrogen atom, is an asymmetric carbon of (R,S) configuration or (R) configuration; and
**(ii)** their addition salts.

2. A compound according to claim 1 wherein R is a methyl group and the asymmetric carbon *C has the (R) configuration.

3. A method of preparing a compound of formula I or one of its addition salts according to claim 1, said method comprising the steps which consist in:
**(i)** deprotecting a compound of the formula 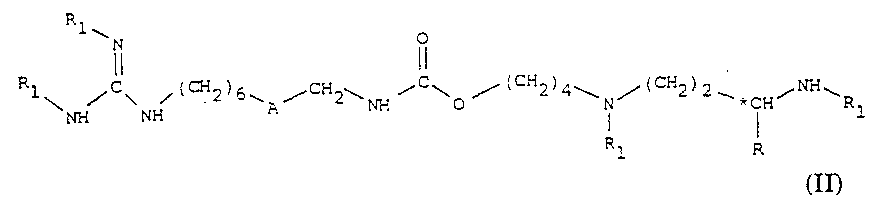 in which:
R is a hydrogen atom or a methyl group,
A is a group -CO-NH- or a group -NH-CO-,
*C, if R is not the hydrogen atom, is an asymmetric carbon of (R,S) or (R) configuration, and at least one of the substituents R₁ is an amino-protecting group of the oxycarbonyl or benzyl type, the other substituents R₁, if appropriate, being a hydrogen atom,
by a treatment adapted to the nature of the amino-protecting group, to give a compound of formula I in the form of the free base or one of its addition salts, and, if necessary,
**(ii)** obtaining the other addition salts from the free base or from its addition salt obtained according to step (i).

4. A method according to claim 3 which also comprises steps selected from the group consisting of:
**(a)** variant A, which comprises the steps consisting in:
**(i)** condensing an alcohol of the formula 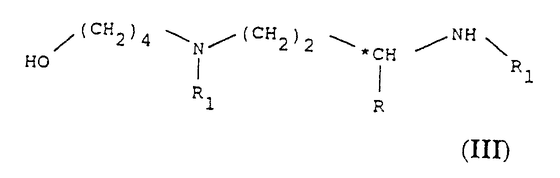 in which:
R is a hydrogen atom or a methyl group,
R₁ is an amino-protecting group, and
*C, if R is not the hydrogen atom, is an asymmetric carbon of (R,S) or (R) configuration,
with a chloroformate or a symmetrical carbonate, in the presence of a base, in an inert solvent, at room temperature (15-25°C), and
**(ii)** reacting the compound obtained in stage (i) with an amine of the formula 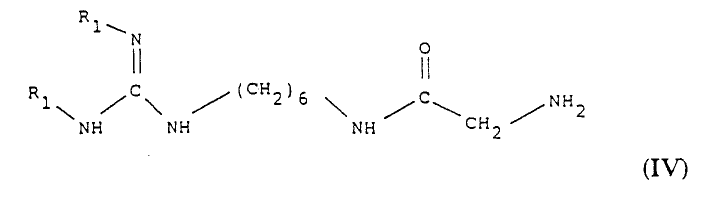 in which R₁ is an amino-protecting group, in an inert solvent, at a temperature of about 25 to 50°C, to give a compound of the formula 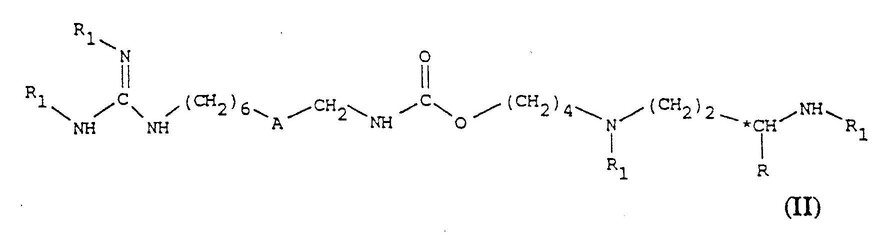 in which:
A is the group -NH-CO-,
R is a hydrogen atom or a methyl group,
*C, if R is not the hydrogen atom, is an asymmetric carbon of (R,S) or (R) configuration, and
R₁ is an amino-protecting group;
and
**(b)** variant B, which comprises the steps consisting in:
**(i)** reacting an acid of the formula 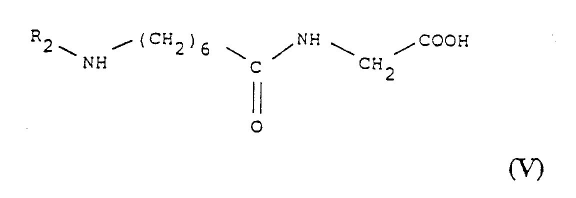 in which R₂ is an amino-protecting group, with diphenylphosphoryl aside of the formula 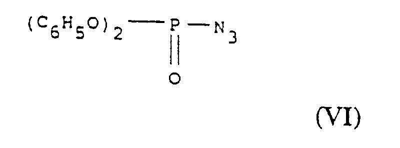 in the presence of a base, in a solvent, at room temperature, to give an intermediate of the formula 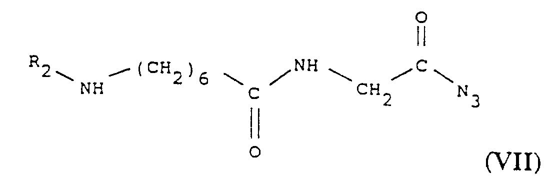
**(ii)** subjecting the compound VII obtained above to a rearrangement known as a Curtius reaction and simultaneously reacting the resulting isocyanate with an alcohol of the formula 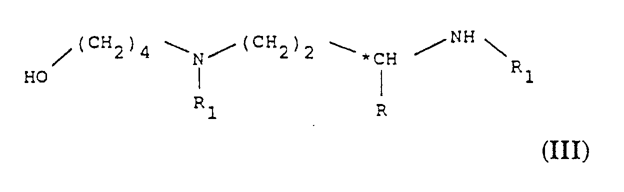 in which:
R is a hydrogen atom or a methyl group,
R₁ is an amino-protecting group differing from the group R₂ above, and
*C, if R is not the hydrogen atom, is an asymmetric carbon of (R,S) or (R) configuration,
in a solvent, at a temperature of about 80 to 140°C, for 5 to 50 hours, to give a compound of the formula 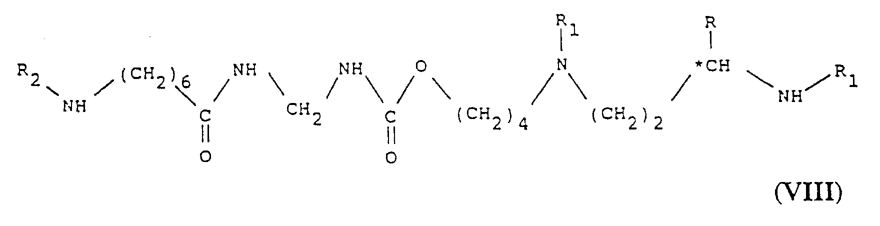 in which R, R₁, R₂ and *C are as defined above,
**(iii)** deprotecting the compound VIII obtained above by a treatment specific for the replacement of the amino-protecting group R₂ with a hydrogen atom, to give a compound of the formula 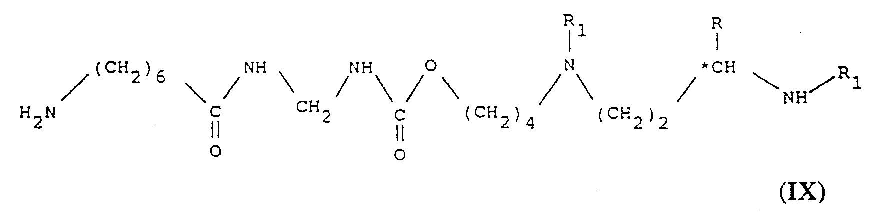 in which R, R₁ and *C are as defined above, and
**(iv)** reacting the compound of formula IX obtained in stage (iii) with aminoiminomethanesulfonic acid, in a solvent, at room temperature (15-25°C), for 8 to 50 hours, to give a compound of the formula 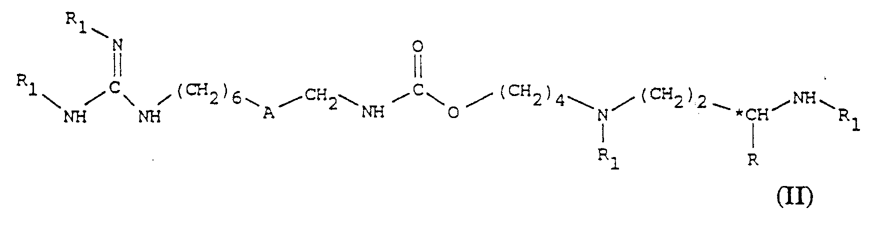 in which:
R and *C are as defined above,
A is the group -CO-NH-, and
R₁ is an amino-protecting group, with the exception of the two groups R₁ carried by the guanidine group, which are each a hydrogen atom, or
**(iv')** as a variant of step (iv) above, reacting the compound of formula IX with a compound of the formula 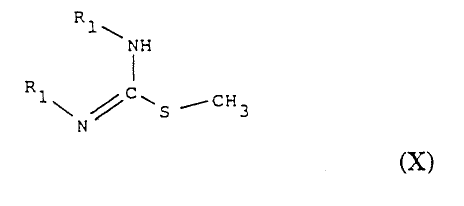 in which:
R₁ is an amino-protecting group, in an inert solvent, especially tetrahydrofuran, in the presence of a base, at room temperature (15-25°C), for 8 to 100 hours, to give a compound of the formula 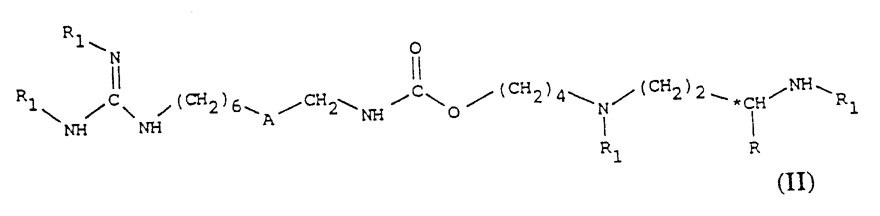
in which:
A is the group -CO-NH-,
R is a hydrogen atom or a methyl group,
R₁ is an amino-protecting group, and
*C, if R is not the hydrogen atom, is an asymmetric carbon of (R,S) or (R) configuration.

5. An intermediate useful in the preparation of a compound of formula I according to claim 1 which is selected from the group consisting of the compounds of the formula 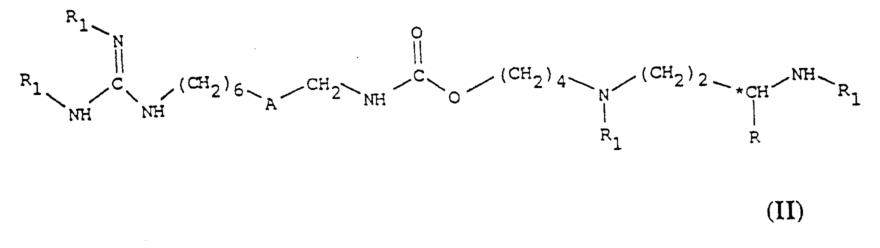 in which:
R is a hydrogen atom or a methyl group,
A is a group -CO-NH- or a group -NH-CO-,
*C, if R is not the hydrogen atom, is an asymmetric carbon of (R,S) configuration or (R) configuration, and
at least one of the substituents R₁ is an amino-protecting group of the oxycarbonyl or benzyl type, the other substituents R₁, if appropriate, being a hydrogen atom.

6. An intermediate useful in the preparation of a compound of formula I according to claim 1 which has the formula 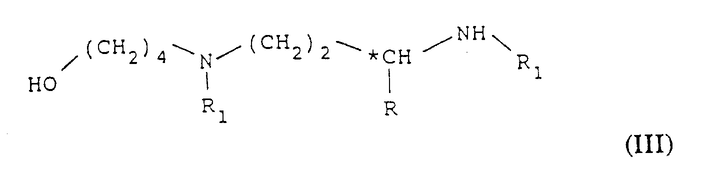 in which:
R is a methyl group,
R₁ is an amino-protecting group, and
*C is an asymmetric carbon of (R,S) or (R) configuration.

7. A use of an immunosuppressive substance selected from the group consisting of the compounds of formula I and their non-toxic addition salts according to claim 1 for the preparation of a drug intended for use in therapeutics to combat immune disorders.

8. A use of a substance selected from the group consisting of the compounds of formula I and their non-toxic addition salts according to claim 1 for the preparation of a drug intended for use in therapeutics to combat malaria.

9. A use of a substance selected from the group consisting of the compounds of formula I and their addition salts according to claim 1 as a pharmacological reagent.

10. A therapeutic composition which contains, in association with a physiologically acceptable excipient, at least one compound selected from the group consisting of the compounds of formula I and their nontoxic addition salts according to claim 1.

## Patentansprüche

1. Verbindung aus der Familie der 15-Deoxyspergualinanaloga,
**dadurch gekennzeichnet**,
daß sie aus der Gruppe ausgewählt ist, bestehend aus:
(i) Verbindungen der Formel: 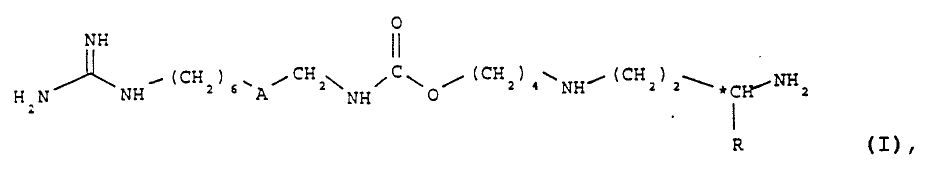 in denen:
A für eine -CO-NH-Gruppe oder eine -NH-CO-Gruppe steht,
R für ein Wasserstoffatom oder eine CH₃-Gruppe steht,
*C, wenn R kein Wasserstoffatom ist, für ein asymmetrisches Kohlenstoffatom der Konfiguration (R,S) oder der Konfiguration (R) steht,
und
(ii) ihren Additionssalzen.

2. Verbindung nach Anspruch 1,
**dadurch gekennzeichnet**,
daß R für eine Methylgruppe steht und das asymmetrische Kohlenstoffatom *C die Konfiguration (R) hat.

3. Verfahren zur Herstellung einer Verbindung der Formel I oder eines ihrer Additionssalze nach Anspruch 1,
wobei dieses Verfahren
**dadurch gekennzeichnet**
ist, daß es die Schritte enthält, bestehend aus:
(i) Entschützen einer Verbindung der Formel: 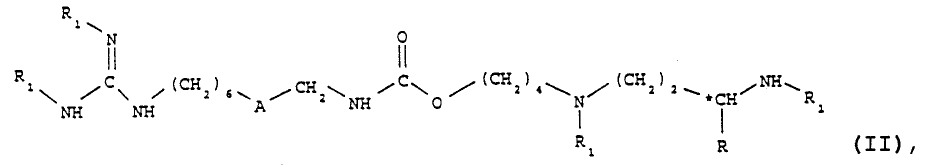 in der:
R für ein Wasserstoffatom oder eine Methylgruppe steht,
A für eine -CO-NH-Gruppe oder eine -NH-CO-Gruppe steht,
*C, wenn R kein Wasserstoffatom ist, für ein asymmetrisches Kohlenstoffatom der Konfiguration (R,S) oder (A) steht,
wobei einer oder mehrere der Substituenten R₁ für eine Amino-Schutzgruppe des Oxycarbonyl- oder Benzyltyps stehen, und die anderen Substituenten R₁ soweit sie vorliegen, für ein Wasserstoffatom stehen,
in einer Behandlung, die an die Art der Amino-Schutzgruppe angepaßt ist, so daß eine Verbindung der Formel I unter Bildung einer freien Base oder eines ihrer Additionssalze erhalten wird,
und, wenn nötig,
(ii) Gewinnung weiterer Additionssalze durch Austausch der freien Base oder eines ihrer Additionssalze, die gemäß Schritt (i) erhalten worden sind.

4. Verfahren nach Anspruch 3,
**dadurch gekennzeichnet**,
daß es ferner die Schritte enthält, die aus der Gruppe ausgewählt sind, bestehend aus:
(a) der Variante A, die die Schritte enthält, bestehend aus:
(i) Kondensation eines Alkohols der Formel: 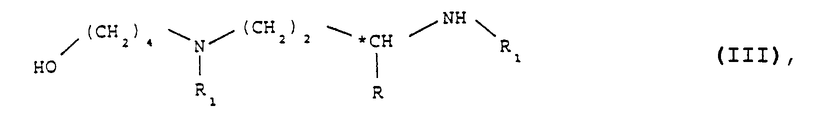 in der:
R für ein Wasserstoffatom oder eine Methylgruppe steht,
R₁ für eine Amino-Schutzgruppe steht,
*C, wenn R kein Wasserstoffatom ist, für ein asymmetrisches Kohlenstoffatom der Konfiguration (R,S) oder (R) steht,
mit einem Chlorformiat oder einem achiralen Kohlensäurederivat bei Zimmertemperatur (15 bis 25°C) in Gegenwart einer Base in einem inerten Lösungsmittel,
(ii) Umsetzen eines Amins der Formel: 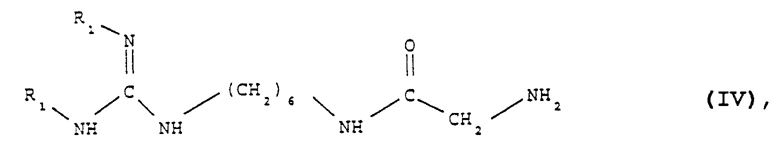 in der R₁ für eine Amino-Schutzgruppe steht, bei einer Temperatur von etwa 25 bis 50°C in einem inerten Lösungsmittel mit der Verbindung, die im Schritt (i) erhalten worden ist, um eine Verbindung der Formel: 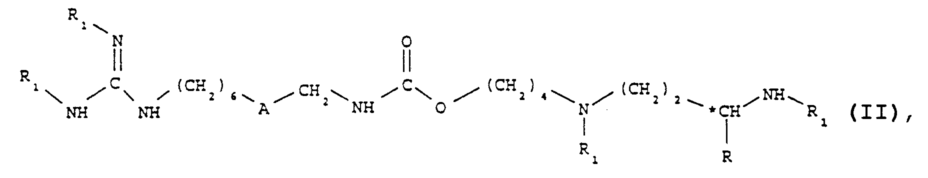 zu erhalten, in der:
A für die -NH-CO-Gruppe steht,
R für ein Wasserstoffatom oder eine Methylgruppe steht,
*C, wenn R kein Wasserstoffatom ist, für ein asymmetrisches Kohlenstoffatom der Konfiguration (R, S) oder (R) steht,
R₁ für eine Amino-Schutzgruppe steht,
(b) die Variante B, die die Schritte enthält, bestehend aus:
(i) Reaktion einer Säure der Formel: 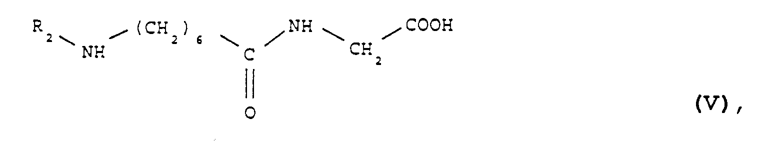 in der R₂ für eine Amino-Schutzgruppe steht, mit Diphenylphosphonsäureazid der Formel: 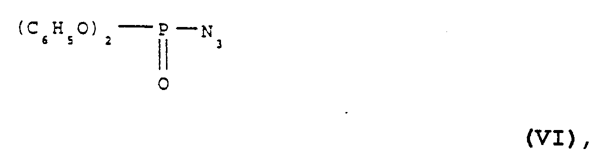 bei Zimmertemperatur in Gegenwart einer Base in einem Lösungsmittel, um eine Zwischenverbindung der Formel: 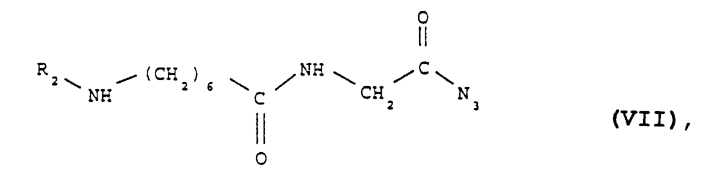 zu erhalten,
(ii) Ausführung einer Umlagerung, die unter dem Namen Curtius-Reaktion bekannt ist, der Verbindung VII, die wie oben erhalten worden ist, und gleichzeitige Reaktion des so erhaltene Isocyanats mit einem Alkohol der Formel: 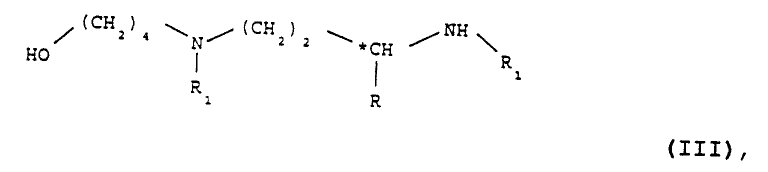 in der:
R für ein Wasserstoffatom oder eine Methylgruppe steht,
R₁ für eine Amino-Schutzgruppe steht, die sich von der obigen Gruppe R₂ unterscheidet,
*C, wenn R kein Wasserstoffatom ist, für ein asymmetrisches Kohlenstoffatom der Konfiguration (R,S) oder (R) steht,
bei einer Temperatur von etwa 80 bis 140°C über einen Zeitraum von 5 bis 50 Stunden in einem Lösungsmittel, um eine Verbindung der Formel: 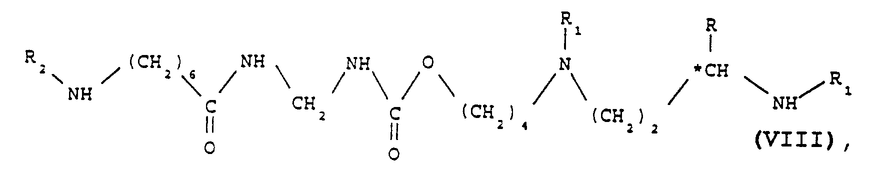 zu erhalten, in der R, R₁, R₂ und *C die gleiche Bedeutung wie oben haben,
(iii) Entschützen der Verbindung VIII, die wie oben erhalten worden ist, nach einem speziellen Verfahren, bei dem die Amino-Schutzgruppe R₂ durch ein Wasserstoffatom ersetzt wird, um eine Verbindung der Formel: 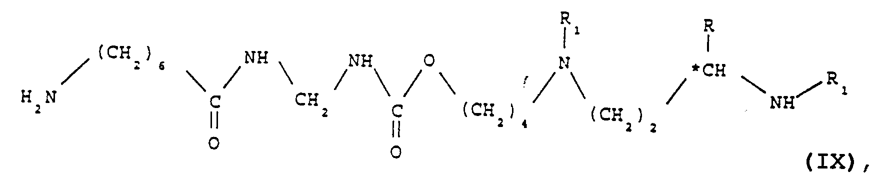 zu erhalten, in der R, R₁ und *C die gleiche Bedeutung wie oben haben,
(iv) Reaktion der Verbindung der Formel IX, die im Schritt (iii) erhalten worden ist, mit Amino-Iminomethansulfonsäure in einem Lösungsmittel über einen Zeitrum von 8 bis 50 Stunden bei Zimmertemperatur (15 bis 25°C), um eine Verbindung der Formel: 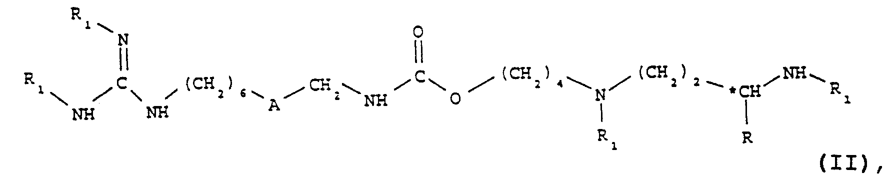 zu erhalten, in der:
R und *C die gleiche Bedeutung wie oben haben,
A für die -CO-NH-Gruppe steht,
R₁ für eine Amino-Schutzgruppe steht, mit Ausnahme der beiden R₁-Gruppen, die an die Guanidin-Funktion gebunden sind, und die jeweils für ein Wasserstoffatom stehen
oder
(iv') Reaktion nach einer Variante des obigen Schrittes (iv) der Verbindung der Formel IX mit einer Verbindung der Formel: 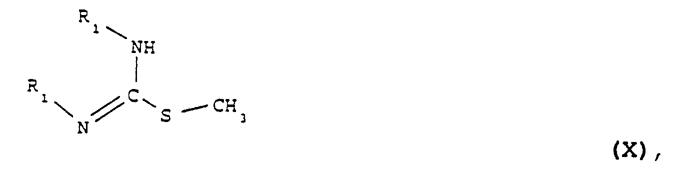 in der:
R₁ für eine Amino-Schutzgruppe steht,
bei Zimmertemperatur (15 bis 25°C) über einen Zeitraum von 8 bis 100 Stunden in Gegenwart einer Base in einem inerten Lösungsmittel, insbesondere Tetrahydrofuran, um eine Verbindung der Formel: 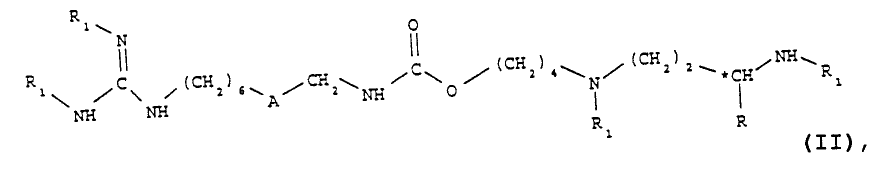 zu erhalten, in der
A für eine -CO-NH-Gruppe steht,
R für ein Wasserstoffatom oder eine Methylgruppe steht,
R₁ für eine Amino-Schutzgruppe steht,
*C, wenn R kein Wasserstoffatom ist, für ein asymmetrisches Kohlenstoffatom der Konfiguration (R,S) oder (R) steht.

5. Zwischenverbindung, die bei der Herstellung einer Verbindung der Formel I nach Anspruch 1 verwendet wird,
**dadurch gekennzeichnet**,
daß sie aus der Gruppe ausgewählt ist, die aus den Verbindungen der Formel: 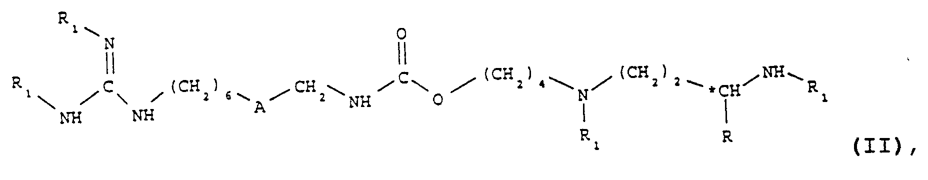 besteht, in der:
R für ein Wasserstoffatom oder eine Methylgruppe steht,
A für eine -CO-NH-Gruppe oder eine -NH-CO-Gruppe steht,
*C, wenn R kein Wasserstoffatom ist, für ein asymmetrisches Kohlenstoffatom der Konfiguration (R,S) oder der Konfiguration (R) steht,
wobei ein oder mehrere Substituenten R₁ für eine AminoSchutzgruppe des Oxycarbonyl- oder Benzyltyps stehen, und die anderen Substituenten R₁, soweit sie vorliegen, für ein Wasserstoffatom stehen.

6. Zwischenverbindung, die bei der Herstellung der Verbindung der Formel I gemäß Anspruch 1 verwendet wird,
**dadurch gekennzeichnet**,
daß sie der Formel: 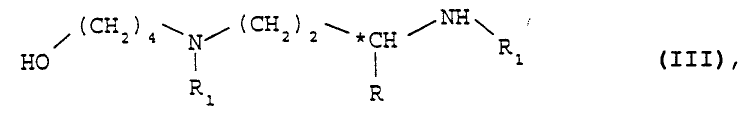 entspricht, in der
R für eine Methylgruppe steht,
R₁ für eine Amino-Schutzgruppe steht,
*C für ein asymmetrisches Kohlenstoffatom der Konfiguration
(R,S) oder (R) steht.

7. Verwendung eines immunsuppressiven Wirkstoffes, ausgewählt aus der Gruppe, die aus den Verbindungen der Formel I und ihren nicht-toxischen Additionssalzen gemäß Anspruch 1 besteht, zur Herstellung eines Arzneimittels zur therapeutischen Behandlung von Immunkrankheiten.

8. Verwendung einer Substanz, ausgewählt aus der Gruppe, die aus den Verbindungen der Formel I und ihren nicht-toxischen Additionssalzen nach Anspruch 1 besteht, zur Herstellung eines Arzneimittel zur therapeutischen Behandlung von Malaria.

9. Verwendung einer Substanz, ausgewählt aus der Gruppe, die aus den Verbindungen der Formel I und ihren Additionssalzen gemäß Anspruch 1 besteht, als pharmakologisches Agens.

10. Wirkstoffzusammensetzung,
**dadurch gekennzeichnet**,
daß sie zusammen mit einem physiologisch unbedenklichen Träger mindestens eine Verbindung enthält, die aus der Gruppe ausgewählt ist, die aus den Verbindungen gemäß Formel I und ihren nichttoxischen Additionssalzen nach Anspruch 1 besteht.
